(19) Europäisches Patentamt
European Patent Office
Office européen des brevets

(11) EP 2 942 045 B1

(12) **EUROPÄISCHE PATENTSCHRIFT**

(45) Veröffentlichungstag und Bekanntmachung des Hinweises auf die Patenterteilung:
**10.10.2018 Patentblatt 2018/41**

(21) Anmeldenummer: **14167167.7**

(22) Anmeldetag: **06.05.2014**

(51) Int Cl.:
***A61K 8/06*** *(2006.01)* ***A61K 8/04*** *(2006.01)*
***A61K 8/02*** *(2006.01)* ***A61K 8/19*** *(2006.01)*
***A61K 8/35*** *(2006.01)* ***A61K 8/81*** *(2006.01)*
***A61Q 19/08*** *(2006.01)* ***A61Q 17/04*** *(2006.01)*

(54) **Perlenförmige kosmetische Darreichungsformen**

Bead-shaped cosmetic dosage forms

Formes galéniques cosmétiques en forme de perle

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(43) Veröffentlichungstag der Anmeldung:
**11.11.2015 Patentblatt 2015/46**

(73) Patentinhaber: **Beiersdorf AG**
**20253 Hamburg (DE)**

(72) Erfinder:
- **Fey, Sven**
  **22397 Hamburg (DE)**
- **Möbius, Janne**
  **20257 Hamburg (DE)**
- **Retzer, Dana**
  **22297 Hamburg (DE)**

(56) Entgegenhaltungen:
**EP-A2- 2 708 264** **WO-A1-03/075861**
**DE-A1- 10 238 450** **DE-A1-102012 211 790**

- **BULE M V ET AL: "Microencapsulation of ubiquinone-10 in carbohydrate matrices for improved stability", CARBOHYDRATE POLYMERS, APPLIED SCIENCE PUBLISHERS, LTD. BARKING, GB, Bd. 82, Nr. 4, 11. November 2010 (2010-11-11), Seiten 1290-1296, XP027266323, ISSN: 0144-8617 [gefunden am 2010-07-13]**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

EP 2 942 045 B1

## Beschreibung

Die vorliegende Erfindung betrifft perlenförmige kosmetische Darreichungsformen, insbesondere solche mit einem Gehalt an stabilisierten stabilisierten oxidationsempfindlichen bzw. UV-empfindlichen Wirkstoffen.

Die vorliegende Erfindung betrifft kosmetische bzw. dermatologische Zubereitungen, enthaltend Wirkstoffe zur Pflege und zum Schutze der Haut, insbesondere der empfindlichen und der trockenen Haut wie auch ganz besonders im Vordergrunde stehend der durch intrinsische und/oder extrinsische Faktoren gealterten oder alternden Haut sowie zur Unterstützung des hauteigenen Lipidmetabolismus sowie die Verwendung solcher Wirkstoffe und Kombinationen solcher Wirkstoffe auf dem Gebiete der kosmetischen und dermatologischen Hautpflege.

Unter kosmetischer Hautpflege ist in erster Linie zu verstehen, dass die natürliche Funktion der Haut als Barriere gegen Umwelteinflüsse (z.B. Schmutz, Chemikalien, Mikroorganismen) und gegen den Verlust von körpereigenen Stoffen (z.B. Wasser, natürliche Fette, Elektrolyte) gestärkt oder wiederhergestellt wird.

Wird diese Funktion gestört, kann es zu verstärkter Resorption toxischer oder allergener Stoffe oder zum Befall von Mikroorganismen und als Folge zu toxischen oder allergischen Hautreaktionen kommen.

Ziel der Hautpflege ist es ferner, den durch tägliche Waschen verursachten Fett- und Wasserverlust der Haut auszugleichen. Dies ist gerade dann wichtig, wenn das natürliche Regenerationsvermögen nicht ausreicht. Außerdem sollen Hautpflegeprodukte vor Umwelteinflüssen, insbesondere vor Sonne und Wind, schützen und die Hautalterung verzögern.

Die chronologische Hautalterung wird z.B. durch endogene, genetisch determinierte Faktoren verursacht. In Epidermis und Dermis kommt es alterungsbedingt z.B. zu folgenden Strukturschäden und Funktionsstörungen, die auch unter den Begriff "Senile Xerosis" fallen können:

a) Trockenheit, Rauhigkeit und Ausbildung von Trockenheitsfältchen,
b) Juckreiz und
c) verminderte Rückfettung durch Talgdrüsen (z.B. nach Waschen).
Exogene Faktoren, wie UV-Licht und chemische Noxen, können kumulativ wirksam sein und z.B. die endogenen Alterungsprozesse beschleunigen bzw. sie ergänzen. In Epidermis und Dermis kommt es insbesondere durch exogene Faktoren z.B. zu folgenden Strukturschäden- und Funktionsstörungen in der Haut, die über Maß und Qualität der Schäden bei chronologischer Alterung hinausgehen:
d) Sichtbare Gefäßerweiterungen (Teleangiektasien, Cuperosis);
e) Schlaffheit und Ausbildung von Falten;
f) lokale Hyper-, Hypo- und Fehlpigmentierungen (z.B. Altersflecken) und
g) vergrößerte Anfälligkeit gegenüber mechanischem Stress (z.B. Rissigkeit).

Die vorliegende Erfindung betrifft insbesondere Produkte zur Pflege der auf natürliche Weise gealterten Haut, sowie zur Behandlung der Folgeschäden der Lichtalterung, insbesondere der unter a) bis g) aufgeführten Phänomene.

Produkte zur Pflege gealterter Haut sind an sich bekannt. Siehe z.B. EP 2 708 264 A2 (PM INTERNAT AG [LU]) 19. März 2014 (2014-03-19). Sie enthalten z.B. Retinoide (Vitamin A-Säure und/oder deren Derivate) bzw. Vitamin A und/oder dessen Derivate. Ihre Wirkung auf die Strukturschäden ist allerdings umfangsmäßig begrenzt. Darüber hinaus gibt es bei der Produktentwicklung erhebliche Schwierigkeiten, die Wirkstoffe in ausreichendem Maße gegen oxidativen Zerfall zu stabilisieren. Die Verwendung Vitamin A-Säure-haltiger Produkte bedingt darüber hinaus oft starke erythematöse Hautreizungen. Retinoide sind daher nur in geringen Konzentrationen einsetzbar.
Insbesondere betrifft die vorliegende Erfindung kosmetische Zubereitungen mit einem wirksamen Schutz vor schädlichen Oxidationsprozessen in der Haut, aber auch zum Schutze kosmetischer Zubereitungen selbst bzw. zum Schutze der Bestandteile kosmetischer Zubereitungen vor schädlichen Oxidationsprozessen.
Die vorliegende Erfindung betrifft ferner Antioxidantien, bevorzugt solche, welche in hautpflegenden kosmetischen oder dermatologischen Zubereitungen eingesetzt werden. Insbesondere betrifft die Erfindung auch kosmetische und dermatologische Zubereitungen, solche Antioxidantien enthaltend. In einer bevorzugten Ausführungsform betrifft die vorliegende Erfindung kosmetische und dermatologische Zubereitungen zur Prophylaxe und Behandlung kosmetischer oder dermatologischer Hautveränderungen wie z.B. der Hautalterung, insbesondere der durch oxidative Prozesse hervorgerufenen Hautalterung.

Weiterhin betrifft die vorliegende Erfindung Wirkstoffe und Zubereitungen, solche Wirkstoffe enthaltend, zur kosmetischen und dermatologischen Behandlung oder Prophylaxe erythematöser, entzündlicher, allergischer oder autoimmunreaktiver Erscheinungen, insbesondere Dermatosen.

Die vorliegende Erfindung betrifft in einer weiteren vorteilhaften Ausführungsform Wirkstoffkombinationen und Zubereitungen, die zur Prophylaxe und Behandlung der lichtempfindlichen Haut, insbesondere von Photodermatosen, dienen.

Die schädigende Wirkung des ultravioletten Teils der Sonnenstrahlung auf die Haut ist allgemein bekannt.

Während Strahlen mit einer Wellenlänge, die kleiner als 290 nm ist (der sogenannte UVC-Bereich), von der Ozonschicht in der Erdatmosphäre absorbiert werden, verursachen Strahlen im Bereich zwischen 290 nm und 320 nm, dem sogenannten UVB-Bereich, ein Erythem, einen einfachen Sonnenbrand oder sogar mehr oder weniger starke Verbrennungen.

Als ein Maximum der Erythemwirksamkeit des Sonnenlichtes wird der engere Bereich um 308 nm angegeben.

Zum Schutz gegen UVB-Strahlung sind zahlreiche Verbindungen bekannt, bei denen es sich um Derivate des 3-Benzylidencamphers, der 4-Aminobenzoesäure, der Zimtsäure, der Salicylsäure, des Benzophenons sowie auch des 2-Phenylbenzimidazols handelt.

Auch für den Bereich zwischen etwa 320 nm und etwa 400 nm, des sogenannten UVA-Bereich, ist es wichtig, Filtersubstanzen zur Verfügung zu haben, da dessen Strahlen Reaktionen bei lichtempfindlicher Haut hervorrufen können. Es ist erwiesen, dass UVA-Strahlung zu einer Schädigung der elastischen und kollagenen Fasern des Bindegewebes führt, was die Haut vorzeitig altern läßt, und dass sie als Ursache zahlreicher phototoxischer und photoallergischer Reaktionen zu sehen ist. Der schädigende Einfluß der UVB-Strahlung kann durch UVA-Strahlung verstärkt werden.

Zum Schutz gegen die Strahlen des UVA-Bereichs werden daher gewisse Derivate des Dibenzoylmethans verwendet, deren Photostabilität (Int. J. Cosm. Science 10, 53 (1988)), nicht in ausreichendem Maße gegeben ist.

Die UV-Strahlung kann aber auch zu photochemischen Reaktionen führen, wobei dann die photochemischen Reaktionsprodukte in den Hautmetabolismus eingreifen.

Vorwiegend handelt es sich bei solchen photochemischen Reaktionsprodukten um radikalische Verbindungen, beispielsweise Hydroxyradikale. Auch undefinierte radikalische Photoprodukte, welche in der Haut selbst entstehen, können aufgrund ihrer hohen Reaktivität unkontrollierte Folgereaktionen an den Tag legen. Aber auch Singulettsauerstoff, ein nichtradikalischer angeregter Zustand des Sauerstoffmoleküls kann bei UV-Bestrahlung auftreten, ebenso kurzlebige Epoxide und viele andere. Singulettsauerstoff beispielsweise zeichnet sich gegenüber dem normalerweise vorliegenden Triplettsauerstoff (radikalischer Grundzustand) durch gesteigerte Reaktivität aus. Allerdings existieren auch angeregte, reaktive (radikalische) Triplettzustände des Sauerstoffmoleküls.

Ferner zählt UV-Strahlung zur ionisierenden Strahlung. Es besteht also das Risiko, dass auch ionische Spezies bei UV-Exposition entstehen, welche dann ihrerseits oxidativ in die biochemischen Prozesse einzugreifen vermögen.

Um diesen Reaktionen vorzubeugen, können den kosmetischen bzw. dermatologischen Formulierungen zusätzliche Antioxidantien und/oder Radikalfänger einverleibt werden.

Es ist bereits vorgeschlagen worden, Vitamin E, eine Substanz mit bekannter antioxidativer Wirkung in Lichtschutzformulierungen einzusetzen, dennoch bleibt auch hier die erzielte Wirkung weit hinter der erhofften zurück.

Aufgabe der Erfindung war es daher auch, kosmetische, dermatologische und pharmazeutische Wirkstoffe und Zubereitungen sowie Lichtschutzformulierungen zu schaffen, die zur Prophylaxe und Behandlung lichtempfindlicher Haut, insbesondere Photodermatosen, bevorzugt PLD dienen.

Weitere Bezeichnungen für die polymorphe Lichtdermatose sind PLD, PLE, Mallorca-Akne und eine Vielzahl von weiteren Bezeichnungen, wie sie in der Literatur (z.B. A. Voelckel et al, Zentralblatt Haut- und Geschlechtskrankheiten (1989), 156, S.2), angegeben sind.

Erythematöse Hauterscheinungen treten auch als Begleiterscheinungen bei gewissen Hauterkrankungen oder -unregelmäßigkeiten auf. Beispielsweise ist der typische Hautausschlag beim Erscheinungsbild der Akne regelmäßig mehr oder weniger stark gerötet.

Hauptsächlich werden Antioxidantien als Schutzsubstanzen gegen den Verderb der sie enthaltenden Zubereitungen verwendet. Dennoch ist bekannt, dass auch in der menschlichen und tierischen Haut unerwünschte Oxidationsprozesse auftreten können. Solche Prozesse spielen eine wesentliche Rolle bei der Hautalterung.

Im Aufsatz "Skin Diseases Associated with Oxidative Injury" in "Oxidative Stress in Dermatology", S. 323 ff. (Marcel Decker Inc., New York, Basel, Hong Kong, Herausgeber: Jürgen Fuchs, Frankfurt, und Lester Packer, Berkeley/Californien), werden oxidative Schäden der Haut und ihre näheren Ursachen aufgeführt.

Auch aus dem Grunde, solchen Reaktionen vorzubeugen, können kosmetischen oder dermatologischen Formulierungen zusätzlich Antioxidantien und/oder Radikalfänger einverleibt werden.

Zwar sind einige Antioxidantien und Radikalfänger bekannt. So ist bereits in den US-Patentschriften 4,144,325 und 4,248,861 sowie aus zahlreichen anderen Dokumenten vorgeschlagen worden, Vitamin E, eine Substanz mit bekannter antioxidativer Wirkung in Lichtschutzformulierungen einzusetzen, dennoch bleibt auch hier die erzielte Wirkung weit hinter der erhofften zurück.

Aufgabe der vorliegenden Erfindung war es somit, Wege zu finden, die die Nachteile des Standes der Technik vermeiden. Insbesondere soll die Wirkung der Behebung der mit der endogenen, chronologischen und exogenen Hautalterung verbundenen Schäden und die Prophylaxe dauerhaft, nachhaltig und ohne das Risiko von Nebenwirkungen sein.

Diesen Übelständen abzuhelfen, war Aufgabe der vorliegenden Erfindung.

Weiterhin sind kosmetische Zubereitungen mit Coenzym Q-10 aus der DE-A-33 09 850 bekannt, die zur Behandlung von Hautkrankheiten, zur Prophylaxe von dystrophischen und dysmetabolischen Zuständen der Haut und zur Anwendung bei chemischen und physikalischen Respirationsschäden oder bei verzögerter Respiration verbunden mit Alter und Abnutzung geeignet sind.

Coenzym Q-10 ist durch folgende Strukturformel gekennzeichnet

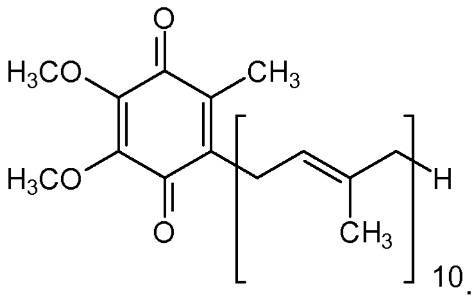

In der japanischen Offenlegungsschrift 58,180,410 ist die Eignung von Coenzym Q-10 für Kosmetika beschrieben. Es soll den Hautzellmetabolismus aktivieren und die Oxidation unterdrücken. Coenzym Q10 hat im Resultat eine wichtige Funktion bei der Prävention von Hautschäden durch UV-Strahlen und der Prävention von Hautalterung. Bei 20 bis 40-jährigen wird die Hautrauhigkeit gebessert, indem der Haut Feuchtigkeit gegeben wird.

Erfindungsgemäß vorteilhaft können die Chinone gewählt werden aus der Gruppe der Biochinone. Biochinone sind prenylierte Chinone, die im Tier- und Pflanzenreich vorkommen und dort biochemische Funktionen erfüllen. Insbesondere bevorzugt sind Ubichinone und Plastochinone.

Erfindungsgemäß vorteilhaft können die Hydrochinone gewählt werden aus der Gruppe der reduzierten Formen der entsprechenden Biochinone, also besonders bevorzugt der Ubichinole und Plastochinole.

Ubichinone stellen die am weitesten verbreiteten u. damit am besten untersuchten Biochinone dar. Ubichinone werden je nach Zahl der in der Seitenkette verknüpften Isopren-Einheiten als Q-1, Q-2, Q-3 usw. oder nach Anzahl der C-Atome als U-5, U-10, U-15 usw. bezeichnet. Sie treten bevorzugt mit bestimmten Kettenlängen auf, z. B. in einigen Mikroorganismen u. Hefen mit n=6. Bei den meisten Säugetieren einschließlich des Menschen überwiegt Q-10.

Ubichinone dienen den Organismen als Elektronenüberträger in der Atmungskette. Sie befinden sich in den Mitochondrien wo sie die cyclische Oxidation und Reduktion der Substrate des Citronensäure-Cyclus ermöglichen.

Plastochinone weisen die allgemeine Strukturformel

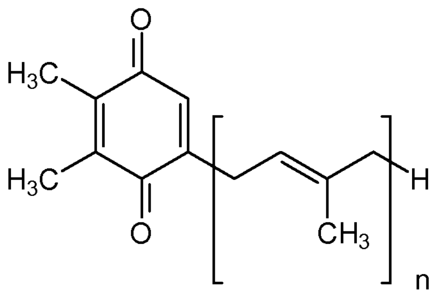

auf. Sie können aus Chloroplasten isoliert werden und spielen als Redoxsubstrate in der Photosynthese beim cyclischen und nichtcyclischen Elektronentransport eine Rolle, wobei sie reversibel in die entsprechenden Hydrochinone (Plastochinol) übergehen. Plastoschinone unterscheiden sich in der Anzahl n der Isopren-Reste und werden endsprechend bezeichnet, z. B. PQ-9 (n=9). Ferner existieren andere Plastochinone mit unterschiedlichen Substituenten am Chinon-Ring.

Ein Übelstand des Standes der Technik besteht darin, dass Ubichinone und Plastochinone in oxidativem Milieu, aber auch unter UV-Strahlungschnell zerfallen und auf diese Weise ihre Wirksamkeit verlieren.

Eine Aufgabe der vorliegenden Erfindung war es daher, die Stabilität von oxidationsempfindlichen bzw. UV-empfindlichen Wirkstoffen zu erhöhen sowie stabile Zubereitungen mit oxidationsempfindlichen bzw. UV-empfindlichen Wirkstoffen zu schaffen, deren Wirksamkeit über einen langen Zeitraum erhalten bleibt.

Flüssigkeiten können bezüglich ihrer rheologischen Eigenschaften durch ihr Fließ- und Deformationsverhalten unterschieden werden. Ideal elastische Körper erleiden durch äußere Kräfte eine elastische Deformation, die bei Wegnahme der äußeren Krafteinwirkung ein spontanes, vollständiges Zurückgehen der Deformation bewirkt. Ideal viskose Körper werden durch äußere Kräfte irreversibel in ihrer Form verändert. Die zunehmende Deformation wird als Fließen bezeichnet. Die meisten Flüssigkeiten sind weder ideal viskos noch ideal elastisch, sondern zeigen sowohl viskose als auch elastische Eigenschaften und werden daher als viskoelastische Substanzen bezeichnet.

Im Großteil viskoelastischer Lösungen werden dispergierte Partikel oder Gasbläschen immer sedimentieren

bzw. aufsteigen. Sie besitzen eine endliche Strukturrelaxationszeit. Das bedeutet, dass die Netzwerke in diesen Systemen auf eine Deformation mit einer entsprechenden Schubspannung reagieren. Diese wird aber in einer endlichen Zeit auf den Wert Null relaxieren, so dass sich die gesamte Lösung wieder in einem stabilen Ruhezustand ohne Spannung befindet. Dies bedeutet weiter, dass diese Lösungen eine definierte Nullviskosität besitzen und somit bei kleinen Scherraten einen konstanten Viskositätswert erreichen.

Im Gegensatz zu diesen Systemen gibt es aber auch solche, in denen dispergierte Partikel oder Gasbläschen nicht sedimentieren. Es fällt auf, dass diese Systeme erst oberhalb eines charakteristischen Werts fließen. Dieser Wert wird Fließgrenze genannt. Bei näherer Betrachtung der rheologischen Eigenschaften dieser Systeme fällt auf, dass der Speichermodul in ganzen Frequenzbereich unabhängig ist von der Oszillationsfrequenz und immer wesentlich größer ist als der Verlustmodul.

Dagegen erreicht der Betrag der komplexen Viskosität auch bei den kleinsten Frequenzen keinen konstanten Wert, sondern steigt weiter an.

Carbopolgele enthalten Acrylsäurepolymere, welche linear oder quervernetzt aufgebaut sein können und die eine hohe Anzahl von Carboxylgruppen tragen. In gelöster Form binden diese Strukturen Wasser. Die Neutralisation der Carboxylgruppen führt aufgrund deren elektrostatischen Abstoßung zu einer Ausdehnung und damit Quellung der Polymerketten. In diesem Zustand erreichen die Carbopolgele ihre typischen rheologischen Eigenschaften wie z.B. die Erhöhung der Viskosität der kosmetischen Zubereitung und/oder Ausbildung einer Fließgrenze.

Der Effekt der Ausbildung einer Fließgrenze beruht somit auf der elektrostatischen Abstoßung der Carboxylgruppen. Zusätzliche Elektrolyte schirmen diese Ladungen ab. Dadurch kollabieren die Netzwerke, die Fließgrenze bricht zusammen, Partikel oder Gasbläschen können nicht mehr in Schwebe gehalten werden.

Unter dem Begriff "Viskosität" versteht man die Eigenschaft einer Flüssigkeit, der gegenseitigen laminaren Verschiebung zweier benachbarter Schichten einen Widerstand (Zähigkeit, innere Reibung) entgegenzusetzen. Man definiert heute diese sogenannte dynamische Viskosität nach $\eta=\tau/D$ als das Verhältnis der Schubspannung zum Geschwindigkeitsgradienten senkrecht zur Strömungsrichtung. Für newtonsche Flüssigkeiten ist $\eta$ bei gegebener Temperatur eine Stoffkonstante mit der SI-Einheit Pascalsekunde (Pa·s).

Der Quotient $v=\eta/\rho$ aus der dynamischen Viskosität $\eta$ und der Dichte $\rho$ der Flüssigkeit wird als kinematische Viskosität v bezeichnet und in der SI-Einheit $m^2/s$ angegeben.

Als Fluidität ($\varphi$) bezeichnet man den Kehrwert der Viskosität ($\varphi=1/\eta$). Bei Salben und dergleichen wird der Gebrauchswert unter anderem mitbestimmt von der sogenannten Zügigkeit. Unter der Zügigkeit einer Salbe oder Salbengrundlage oder dergleichen versteht man deren Eigenschaft, beim Abstechen verschieden lange Fäden zu ziehen; dementsprechend unterscheidet man kurz- und langzügige Stoffe.

Während die graphische Darstellung des Fließverhaltens newtonscher Flüssigkeiten bei gegebener Temperatur eine Gerade ergibt, zeigen sich bei den sogenannten nichtnewtonschen Flüssigkeiten in Abhängigkeit vom jeweiligen Geschwindigkeitsgefälle D (Schergeschwindigkeit $\dot{\gamma}$) bzw. der Schubspannung $\tau$ oft erhebliche Abweichungen. In diesen Fällen läßt sich die sogenannte scheinbare Viskosität bestimmen, die zwar nicht der Newtonschen Gleichung gehorcht, aus der sich jedoch durch graphische Verfahren die wahren Viskositätswerte ermitteln lassen.

Die Fallkörperviskosimetrie ist lediglich zur Untersuchung newtonscher Flüssigkeiten sowie von Gasen geeignet. Sie basiert auf dem Stokes-Gesetz, nach dem für das Fallen einer Kugel durch eine sie umströmende Flüssigkeit die dynamische Viskosität $\eta$ aus

$$\eta = \frac{2r^2(\rho_K - \rho_{Fl})\cdot g}{9\cdot v}$$

bestimmbar ist, wobei
r = Radius der Kugel, v = Fallgeschwindigkeit, $\rho_K$ = Dichte der Kugel, $\rho_{Fl}$ = Dichte der Flüssigkeit und g = Fallbeschleunigung.

Carbopolgele enthalten Acrylsäurepolymere, welche linear oder quervernetzt aufgebaut sein können und die eine hohe Anzahl von Carboxylgruppen tragen. In gelöster Form binden diese Strukturen Wasser. Die Neutralisation der Carboxylgruppen führt aufgrund deren elektrostatischen Abstoßung zu einer Ausdehnung und damit Quellung der Polymerketten. In diesem Zustand erreichen die Carbopolgele ihre typischen rheologischen Eigenschaften wie z.B. die Erhöhung der Viskosität der kosmetischen Zubereitung und/oder Ausbildung einer Fließgrenze.
Der Effekt der Ausbildung einer Fließgrenze beruht somit auf der elektrostatischen Abstoßung der Carboxylgruppen. Zusätzliche Elektrolyte schirmen diese Ladungen ab. Dadurch kollabieren die Netzwerke, die Fließgrenze bricht zusammen, Partikel oder Gasbläschen können nicht mehr in Schwebe gehalten werden.
Eine weiterer Aspekt der vorliegenden Erfindung bestand folglich darin, diesen Nachteilen entgegenzuwirken.
Es war überraschend und für den Fachmann nicht vorauszusehen, und darin liegt die Lösung der Aufgaben erfindungsgemäß begründet, dass

im Wesentlichen kugelförmige Partikel, deren mittlerer Durchmesser gewählt wird aus dem Bereich von 0,1 bis 10 mm, bevorzugt 2 bis 7,5 mm, und welche umfassen:

- eine Polymermatrix, welche im Wesentlichen unlöslich in Wasser ist,
- eine oder mehrere Substanzen gewählt aus der Gruppe der Ubichinone und/oder Plastochinone
- eine oder mehrere Lichtfiltersubstanzen, welche gewählt wird oder werden aus der Gruppe der Metalloxidpigmente,
- gegebenenfalls eine oder mehrere Lichtfiltersubstanzen, welche gewählt wird oder werden aus der Gruppe der üblichen kosmetischen Lichtschutzfiltersubstanzen den Übelständen des Standes der Technik abhelfen.

Die erfindungsgemäßen Zubereitungen stellen sowohl für sich genommen interessante kosmetische Präparate der, mittels derer die eingearbeiteten Substanzen gewählt aus der Gruppe der Ubichinone und/oder Plastochinone vorzüglich gegen den Zutritt von Luftsauerstoff und schädliche Einwirkung von sichtbarem und ultraviolettem Licht geschützt sind, Siei lassen sich auch ihrerseits wiederum in andere kosmetische oder dermatologische Zubereitungen einarbeiten, insbesondere solche, welche sich durch eine gewisssen Viskosität auszeichnen.

Besonders vorteilhafte Ausführungsformen, siehe insbesondere Anspruch 1, sind daher kosmetische Zubereitungen, umfassend

- eine äußere fließfähige gelartige Phase auf wäßriger Basis
  - welche eine Transmission für Licht der Wellenlänge von 700 nm aus dem Bereich von 30 bis 100 % aufweist
  - welche gegebenenfalls übliche, in Wasser lösliche oder dispergierbare Hilfs- und oder Zusatzstoffe enthält,
- eine innere feste partikuläre Phase, welche
  - im wesentlichen kugelförmige Partikel umfaßt, deren mittlerer Durchmesser gewählt wird aus dem Bereich von 0,1 bis 10 mm, bevorzugt 2 bis 7,5 mm, und wobei die partikuläre Phase umfaßt
    - eine Polymermatrix, welche im wesentlichen unlöslich in Wasser ist,
    - eine oder mehrere Substanzen gewählt aus der Gruppe der Ubichinone und/oder Plastochinone
    - eine oder mehrere Lichtfiltersubstanzen, welche gewählt wird oder werden aus der Gruppe der Metalloxidpigmente,
    - gegebenenfalls eine oder mehrere Lichtfiltersubstanzen, welche gewählt wird oder werden aus der Gruppe der üblichen kosmetischen Lichtschutzfiltersubstanzen weitere wesentliche Merkmale der beanspruchten kosmetischen Zubereitung sind:
- eine äußere fließfähige gelartige Phase auf wäßriger Basis
  - welche eine Viskosität gewählt aus dem Intervall von 1000 - 10000 mPas bei einer Temperatur von 25 °C und einer Scherrate von ca. 10, vorzugsweise von von 3000 - 9000 bei einer Temperatur von 25 °C und einer Scherrate von ca. 10, insbesondere bevorzugt von 5000 -8000 bei einer Temperatur von 25 °C und einer Scherrate von ca. 10 aufweist,
    und
  - welche gegebenenfalls übliche, in Wasser lösliche oder dispergierbare Hilfs- und oder Zusatzstoffe enthält,
- eine innere feste partikuläre Phase, welche
  - im wesentlichen kugelförmige Partikel umfaßt, deren mittlerer Durchmesser gewählt wird aus dem Bereich von 0,1 bis 10 mm, bevorzugt 2 bis 7,5 mm, und wobei die partikuläre Phase umfaßt
    - eine Polymermatrix, welche im wesentlichen unlöslich in Wasser ist,
    - eine oder mehrere Substanzen gewählt aus der Gruppe der Ubichinone und/oder Plastochinone
    - eine oder mehrere Lichtfiltersubstanzen, welche gewählt wird oder werden aus der Gruppe der Metalloxidpigmente,
    - gegebenenfalls eine oder mehrere Lichtfiltersubstanzen, welche gewählt wird oder werden aus der Gruppe der üblichen kosmetischen Lichtschutzfiltersubstanzen

Auch diese Zubereitungen stellen in jeglicher Hinsicht überaus befriedigende Präparate dar, welche nicht auf eine eingeschränkte Rohstoffauswahl begrenzt sind. Dementsprechend eignen sie sich ganz besonders, um als Grundlage für Zubereitungsformen mit vielfältigen Anwendungszwecken zu dienen. Die erfindungsgemäßen Zubereitungen zeigen sehr gute sensorische und kosmetische Eigenschaften, wie beispielsweise die Verteilbarkeit auf der Haut oder das Einzugsvermögen in die Haut, und zeichen sich ferner durch eine sehr gute Lichtschutzeffektivität bei gleichzeitig hervorragenden Hautpflegedaten aus.

Bei einer Lichtlagerung einer unverkapselten und lichtfilterfreien Emulsion wird der Wirkstoff nahezu vollständig über die Zeit abgebaut. Liegen diese Wirkstoffe hingegen in einer Mischung mit Lichtfiltern in verkapselter Form vor ist der Abbau deutlich abgeschwächt und der Wirkstoff auch nach einer längeren Lichtlagerung zu einem hohen Prozentsatz nachzuweisen.

Tabelle 1

| Prüfmuster | Q10 [%] | | | |
|---|---|---|---|---|
| | unbestrahlt | temp Kontrolle * | 24 h | 48 h |
| A. ISP Captivates GL 7796 | 0,24 | 0,23 | 0,21 (87 %) | 0,16(66%) |
| B. KONLONFREE 48 | 0,06 | 0,06 | 0,001 (1,4%) | -- |
| Prüfmuster A: verkapselte Emulsion mit TiO2<br>Prüfmuster B: unverkapselte Emulsion ohne Lichtfilter | | | | |

Tabelle 2

| Prüfmuster | Q10 [%] | | |
|---|---|---|---|
| | temp Kontrolle * | 24 h | 48 h |
| 1. ISP Captiv. GL7827 | 0,145 | 0,039 (27 %) | 0,016 (11 %) |
| 2. ISP Captiv. GL 7834 | 0,170 | 0,010 (6 %) | 0,004 (3 %) |
| 3. ISP Captiv. GL 7827 in Gel 85 | 0,105 | 0,049 (47 %) | 0,035 (33 %) |
| 4. ISP Captiv. GL 7834 in Gel 85 | 0,076 | 0,010 (13 %) | 0,006 (7 %) |
| Prüfmuster 1: verkapselte Emulsion mit hohem Titandioxid Anteil<br>Prüfmuster 2: verkapselte Emulsion mit geringerem Titandioxid Anteil<br>Prüfmuster 3: verkapselte Emulsion analog Prüfmuster 1 mit hohem Titandioxid Anteil, Perlen in Trägergel<br>Prüfmuster 4: verkapselte Emulsion analog Prüfmuster 2 mit geringerem Titandioxid Anteil, Perlen in Trägergel | | | |

Erfindungsgemäß vorteilhafte Ausführungsformen der vorliegenden Erfindung können erhalten werden, indem die äußere fließfähige gelartige Phase auf wäßriger Basis einen Gehalt an einem oder mehreren Hydrokolloiden aufweist, in welche die erfindungsgemäßen, im wesentlichen kugelförmigen Partikel eingearbeitet werden.

"Hydrokolloid" ist die technologische Kurzbezeichnung für die an sich richtigere Bezeichnung "hydrophiles Kolloid". Hydrokolloide sind Makromoleküle, die eine weitgehend lineare Gestalt haben und über intermolekulare Wechselwirkungskräfte verfügen, die Neben- und Hauptvalenzbindungen zwischen den einzelnen Molekülen und damit die Ausbildung eines netzartigen Gebildes ermöglichen. Sie sind teilweise wasserlösliche natürliche oder synthetische Polymere, die in wässrigen Systemen Gele oder viskose Lösungen bilden. Sie erhöhen die Viskosität des Wassers, indem sie entweder Wassermoleküle binden (Hydratation) oder aber das Wasser in ihre unter sich verflochtenen Makromoleküle aufnehmen und einhüllen, wobei sie gleichzeitig die Beweglichkeit des Wassers einschränken. Solche wasserlöslichen Polymere stellen eine große Gruppe chemisch sehr unterschiedlicher natürlicher und synthetischer Polymere dar, deren gemeinsames Merkmal ihre Löslichkeit in Wasser bzw. wäßrigen Medien ist. Voraussetzung dafür ist, dass diese Polymere über eine für die Wasserlöslichkeit ausreichende Anzahl an hydrophilen Gruppen besitzen und nicht zu stark vernetzt sind. Die hydrophilen Gruppen können nichtionischer, anionischer oder kationischer Natur sein, beispielsweise wie folgt:

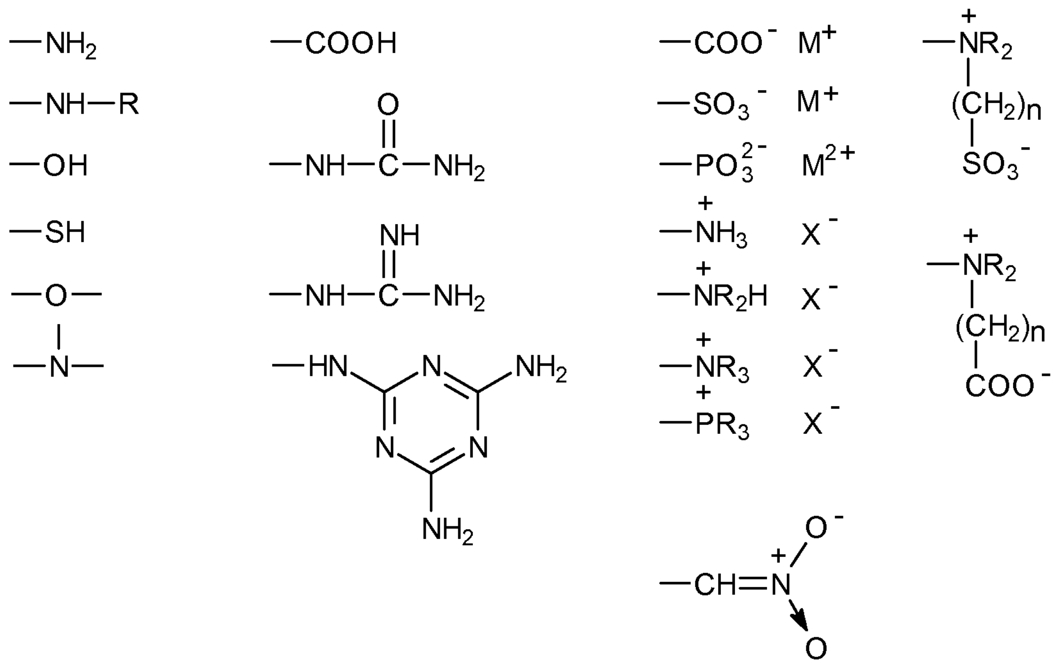

Die Gruppe der kosmetisch und dermatologisch relevanten Hydrokolloide läßt sich wie folgt einteilen in:

- organische, natürliche Verbindungen, wie beispielsweise Agar-Agar, Carrageen, Tragant, Gummi arabicum, Alginate, Pektine, Polyosen, Guar-Mehl, Johannisbrotbaumkernmehl, Stärke, Dextrine, Gelatine, Casein,
- organische, abgewandelte Naturstoffe, wie z. B. Carboxymethylcellulose und andere Celluloseether, Hydroxyethyl- und -propylcellulose und dergleichen,
- organische, vollsynthetische Verbindungen, wie z. B. Polyacryl- und Polymethacryl-Verbindungen, Vinylpolymere, Polycarbonsäuren, Polyether, Polyimine, Polyamide,
- anorganische Verbindungen, wie z. B. Polykieselsäuren, Tonmineralien wie Montmorillonite, Zeolithe, Kieselsäuren.

Erfindungsgemäß bevorzugte Hydrokolloide sind beispielsweise Methylcellulosen, als welche die Methylether der Cellulose bezeichnet werden. Sie zeichnen sich durch die folgende Strukturformel aus

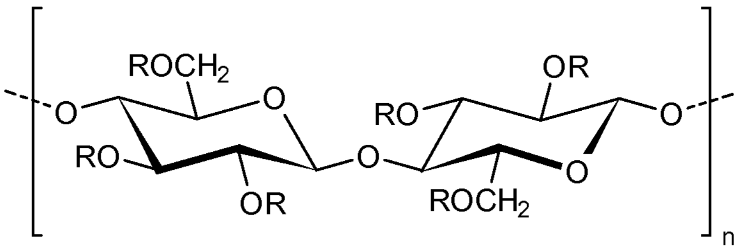

Strukturformel I

in der R ein Wasserstoff oder eine Methylgruppe darstellen kann.

Insbesondere vorteilhaft im Sinne der vorliegenden Erfindung sind die im allgemeinen ebenfalls als Methylcellulosen bezeichneten Cellulosemischether, die neben einem dominierenden Gehalt an Methyl- zusätzlich 2-Hydroxyethyl-, 2-Hydroxypropyl- oder 2-Hydroxybutyl-Gruppen enthalten. Besonders bevorzugt sind (Hydroxypropyl)methylcellulosen, beispielsweise die unter der Handelsbezeichnung Methocel E4M bei der Dow Chemical Comp. erhältlichen.

Erfindungsgemäß ferner vorteilhaft ist Natriumcarboxymethylcellulose, das Natrium-Salz des Glykolsäureethers der Cellulose, für welches R in Strukturformel I ein Wasserstoff und/oder $CH_2$-COONa darstellen kann. Besonders bevorzugt ist die unter der Handelsbezeichnung Natrosol Plus 330 CS bei Aqualon erhältliche, auch als Cellulose Gum bezeichnete Natriumcarboxymethylcellulose.

Besonders bevorzugt im Sinne der vorliegenden Erfindung ist ferner Xanthan (CAS-Nr. 11138-66-2), auch Xanthan Gummi genannt, welches ein anionisches Heteropolysaccharid ist, das in der Regel durch Fermentation aus Maiszucker gebildet und als Kaliumsalz isoliert wird. Es wird von Xanthomonas campestris und einigen anderen Species unter aeroben Bedingungen mit einem Molekulargewicht von $2 \times 10^6$ bis $24 \times 10^6$ produziert. Xanthan wird aus einer Kette mit $\beta$-1,4-gebundener Glucose (Cellulose) mit Seitenketten gebildet. Die Struktur der Untergruppen besteht aus Glucose, Mannose, Glucuronsäure, Acetat und Pyruvat. Xanthan ist die Bezeichnung für das erste mikrobielle anionische Heteropolysaccharid. Es wird von Xanthomonas campestris und einigen anderen Species unter aeroben Bedingungen mit einem Molekulargewicht von 2-15 $10^6$ produziert. Xanthan wird aus einer Kette mit $\beta$-1,4-gebundener Glucose (Cellulose) mit Seitenketten gebildet. Die Struktur der Untergruppen besteht aus Glucose, Mannose, Glucuronsäure, Acetat und Pyruvat. Die Anzahl der Pyruvat-Einheiten bestimmt die Viskosität des Xanthans. Xanthan wird in zweitägigen Batch-Kulturen mit einer Ausbeute von 70-90 %, bezogen auf eingesetztes Kohlenhydrat, produziert. Dabei werden Ausbeuten von 25-30 g/l erreicht. Die Aufarbeitung erfolgt nach Abtöten der Kultur durch Fällung mit z. B. 2-Propanol. Xanthan wird anschließend getrocknet und gemahlen.

Ein besonders vorteilhafter Gelbildner im Sinne der vorliegenden Erfindung ist ferner Carrageen, ein gelbildender und ähnlich wie Agar aufgebauter Extrakt aus nordatlant., zu den Florideen zählenden Rotalgen (Chondrus crispus u. Gigartina stellata).

Häufig wird die Bezeichnung Carrageen für das getrocknete Algenprodukt und Carrageenan für den Extrakt aus diesem verwendet. Das aus dem Heißwasserextrakt der Algen ausgefällte Carrageen ist ein farbloses bis sandfarbenes Pulver mit einem Molekulargewichtsbereich von 100 000-800 000 und einem Sulfat-Gehalt von ca. 25 %. Carrageen, das in warmem Wasser sehr leicht lösl. ist; beim Abkühlen bildet sich ein thixotropes Gel, selbst wenn der Wassergehalt 95-98 % beträgt. Die Festigkeit des Gels wird durch die Doppelhelix-Struktur des Carrageens bewirkt. Beim Carrageenan unterscheidet man drei Hauptbestandteile: Die gelbildende $\kappa$-Fraktion besteht aus D-Galactose-4-sulfat und 3,6-Anhydro-$\alpha$-D-galactose, die abwechselnd in 1,3- und 1,4-Stellung glykosidisch verbunden sind (Agar enthält demgegenüber 3,6-Anhydro-$\alpha$-L-galactose). Die nicht gelierende $\lambda$-Fraktion ist aus 1,3-glykosidisch verknüpften D-Galactose-2-sulfat und 1,4-verbundenen D-Galactose-2,6-disulfat-Resten zusammengesetzt u. in kaltem Wasser leicht löslich. Das aus D-Galactose-4-sulfat in 1,3-Bindung und 3,6-Anhydro-$\alpha$-D-galactose-2-sulfat in 1,4-Bindung aufgebaute $\iota$-Carrageenan ist sowohl wasserlöslich als auch gelbildend. Weitere Carrageen-Typen werden ebenfalls mit griechischen Buchstaben bezeichnet: $\alpha$, $\beta$, $\gamma$, $\mu$, v, $\xi$, $\pi$, $\omega$, $\chi$. Auch die Art vorhandener Kationen ($K^+$, $NH_4^+$, $Na^+$, $Mg^{2+}$, $Ca^{2+}$) beeinflußt die Löslichkeit der Carrageene.

Polyacrylate sind ebenfalls vorteilhaft im sinne der vorliegenden Erfindung zu verwendende Gelatoren. Erfindungsgemäß vorteilhafte Polyacrylate sind Acrylat-Alkylacrylat-Copolymere, insbesondere solche, die aus der Gruppe der sogenannten Carbomere oder Carbopole (Carbopol® ist eigentlich eine eingetragene Marke der B. F. Goodrich Company) gewählt werden. Insbesondere zeichnen sich das oder die erfindungsgemäß vorteilhaften Acrylat-Alkylacrylat-Copolymere durch die folgende Struktur aus:

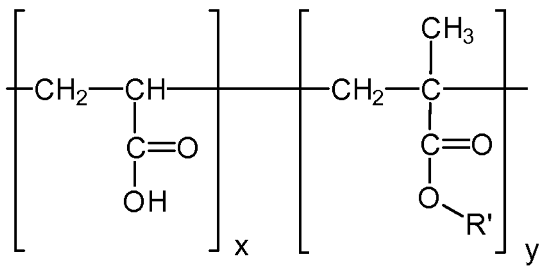

Darin stellen R' einen langkettigen Alkylrest und x und y Zahlen dar, welche den jeweiligen stöchiometrischen Anteil der jeweiligen Comonomere symbolisieren.

Erfindungsgemäß besonders bevorzugt sind Acrylat-Copolymere und/oder Acrylat-Alkylacrylat-Copolymere, welche unter den Handelbezeichnungen Carbopol® 1382, Carbopol® 981 und Carbopol® 5984 von der B. F.Goodrich Company erhältlich sind.

Ferner besonders vorteilhaft sind Copolymere aus $C_{10-30}$-Alkylacrylaten und einem oder mehreren Monomeren der Acrylsäure, der Methacrylsäure oder deren Ester, die kreuzvernetzt sind mit einem Allylether der Saccharose oder einem Allylether des Pentaerythrit.

Besonders vorteilhaft sind Verbindungen, die die INCI-Bezeichnung "Acrylates/C 10-30 Alkyl Acrylate Crosspolymer" tragen. Insbesondere vorteilhaft sind die unter den Handelsbezeichnungen Pemulen TR1 und Pemulen TR2 bei der B. F. Goodrich Company erhältlichen.

Ferner besonders vorteilhaft sind Ammoniumacryloyldimethyltaurate/Vinylpyrrolidoncopolymere, insbesondere solche, die die Summenformel $[C_7H_{16}N_2SO_4]_n$ $[C_6H_9NO]_m$ auf, einer statistischen Struktur wie folgt entsprechend

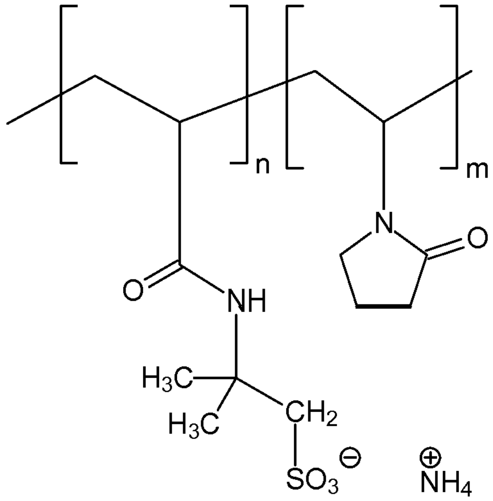

Bevorzugte Spezies im Sinne der vorliegenden Erfindung sind in den Chemical Abstracts unter den Registraturnummern 58374-69-9, 13162-05-5 und 88-12-0 abgelegt und erhältlich unter der Handelsbezeichnung Aristoflex® AVC der Gesellschaft Clariant GmbH.
Die Gesamtmenge an einem oder mehreren Hydrokolloiden wird in den fertigen kosmetischen oder dermatologischen Zubereitungen erfindungsgemäß vorteilhaft aus dem Bereich von 0,005 bis 5 Gew.-%, bevorzugt zwischen 0,1 und 2,0 Gew.-%, insbesondere zwischen 0,25 und 0,75 Gew.-% gewählt, jeweils bezogen auf das Gesamtgewicht der äußeren fließfähigen gelartigen Phase auf wäßriger Basis.

Die Polymermatrix, die gleichsam das Grundgerüst der Partikulären Phase darstellt, kann gewählt werden aus der Gruppe der üblichen, kosmetisch oder dermatologisch unbedenklichen, im wesentlichen wasserunlöslichen Polymersubstanzen, beispielsweise gewählt aus der Gruppe der folgenden Substanzen: Algin, Carrageen, Agar, Gellan Gum, Chitosan. Vorteilhaft ist insbesondere, eine Matrix aus Alginaten, vorzugsweise Natriumalginaten, wie sie auch in der DE 44 24 998 A1 verwendet werden.

Erfindungsgemäß bevorzugte Metalloxidpigmente sind insbesondere Oxide des Titans ($TiO_2$), Zinks (ZnO), Eisens (z. B. $Fe_2O_3$), Zirkoniums ($ZrO_2$), Siliciums ($SiO_2$), Mangans (z. B. MnO), Aluminiums ($Al_2O_3$), Cers (z. B. $Ce_2O_3$), Mischoxide der entsprechenden Metalle sowie Abmischungen aus solchen Oxiden sowie das Sulfat des Bariums ($BaSO_4$).

Ganz besonders vorteilhaft ist es, Oxide des Titans ($TiO_2$) als erfindungsgemäß besonders bevorzugte Metalloxidpigmente einzusetzen.

Die Pigmente können vorteilhaft im Sinne der vorliegenden Erfindung auch in Form kommerziell erhältlicher öliger oder wäßriger Vordispersionen zur Anwendung kommen. Diesen Vordispersionen können vorteilhaft Dispergierhilfsmittel und/oder Solubilisationsvermittler zugesetzt sein.

Die Pigmente können erfindungsgemäß vorteilhaft oberflächlich behandelt ("gecoatet") sein, wobei beispielsweise ein hydrophiler, amphiphiler oder hydrophober Charakter gebildet werden bzw. erhalten bleiben soll. Diese Oberflächenbehandlung kann darin bestehen, dass die Pigmente nach an sich bekannten Verfahren mit einer dünnen hydrophilen und/oder hydrophoben anorganischen und/oder organischen Schicht versehen werden. Die verschiedenen Oberflächenbeschichtungen können im Sinne der vorliegenden Erfindung auch Wasser enthalten.

Anorganische Oberflächenbeschichtungen im Sinne der vorliegenden Erfindung können bestehen aus Aluminiumoxid ($Al_2O_3$), Aluminiumhydroxid $Al(OH)_3$, bzw. Aluminiumoxidhydrat (auch: Alumina, CAS-Nr.: 1333-84-2), Natriumhexametaphosphat $(NaPO_3)_6$, Natriummetaphosphat $(NaPO_3)_n$, Siliciumdioxid ($SiO_2$) (auch: Silica, CAS-Nr.: 7631-86-9), oder Eisenoxid ($Fe_2O_3$). Diese anorganischen Oberflächenbeschichtungen können allein, in Kombination und/oder in Kombination mit organischen Beschichtungsmaterialien vorkommen.

Organische Oberflächenbeschichtungen im Sinne der vorliegenden Erfindung können bestehen aus pflanzlichem oder tierischem Aluminiumstearat, pflanzlicher oder tierischer Stearinsäure, Laurinsäure, Dimethylpolysiloxan (auch: Dimethicone), Methylpolysiloxan (Methicone), Simethicone (einem Gemisch aus Dimethylpolysiloxan mit einer durchschnittlichen Kettenlänge von 200 bis 350 Dimethylsiloxan-Einheiten und Silicagel) oder Alginsäure. Diese organischen Oberflächenbeschichtungen können allein, in Kombination und/oder in Kombination mit anorganischen Beschichtungsmaterialien vorkommen.

Erfindungsgemäß geeignete Zinkoxidpartikel und Vordispersionen von Zinkoxidpartikeln sind unter folgenden Handelsbezeichnungen bei den aufgeführten Firmen erhältlich:

| Handelsname | Coating | Hersteller |
|---|---|---|
| Z- Cote HP1 | 2% Dimethicone | BASF |
| Z- Cote | / | BASF |
| ZnO NDM | 5% Dimethicone | H&R |
| ZnO Neutral | / | H&R |
| MZ 505 M | 5% Methicone | Tayca Corp. |

Geeignete Titandioxidpartikel und Vordispersionen von Titandioxidpartikeln sind unter folgenden Handelsbezeichnungen bei den aufgeführten Firmen erhältlich:

| Handelsname | Coating | Hersteller |
|---|---|---|
| MT-100TV | Aluminiumhydroxid / Stearinsäure | Tayca Corporation |
| MT-100Z | Aluminiumhydroxid / Stearinsäure | Tayca Corporation |
| Eusolex T-2000 | Alumina / Simethicone | Merck KgaA |
| Titandioxid T805 (Uvinul $TiO_2$) | Octyltrimethylsilan | Degussa |
| Tioveil AQ 10PG | Alumina / Silica | Solaveil / Uniquema |

| | | |
|---|---|---|
| A310Tudor Aspen | - | Kingfisher Colours Ltd. |
| Kronos 1171 | - | Kronos Worldwide, Inc. |

Erfindungsgemäß vorteilhafte UV-Filtersubstanzen sind beispielsweise Dibenzoylmethanderivate, insbesondere das 4-(tert.-Butyl)-4'-methoxydibenzoylmethan (CAS-Nr. 70356-09-1), welches von DSM unter der Marke Parsol® 1789 und von Merck unter der Handelsbezeichnung Eusolex® 9020 verkauft wird.

Vorteilhafte weitere UV-Filtersubstanzen im Sinne der vorliegenden Erfindung sind sulfonierte, wasserlösliche UV-Filter, wie z. B.

- Phenylen-1,4-bis-(2-benzimidazyl)-3,3'-5,5'-tetrasulfonsäure und ihre Salze, besonders die entsprechenden Natrium-, Kalium- oder Triethanolammonium-Salze, insbesondere das Phenylen-1,4-bis-(2-benzimidazyl)-3,3'-5,5'-tetrasulfonsäure-bis-natriumsalz mit der INCI-Bezeichnung Bisimidazylate (CAS-Nr.: 180898-37-7), welches beispielsweise unter der Handelsbezeichnung Neo Heliopan AP bei Haarmann & Reimer erhältlich ist;
- Salze der 2-Phenylbenzimidazol-5-sulfonsäure, wie ihr Natrium-, Kalium- oder ihr Triethanolammonium-Salz sowie die Sulfonsäure selbst mit der INCI Bezeichnung Phenylbenzimidazole Sulfonsäure (CAS.-Nr. 27503-81-7), welches beispielsweise unter der Handelsbezeichnung Eusolex 232 bei Merck oder unter Neo Heliopan Hydro bei Haarmann & Reimer erhältlich ist;
- 1,4-di(2-oxo-10-Sulfo-3-bornylidenmethyl)-Benzol (auch: 3,3'-(1,4-Phenylendimethylene)-bis-(7,7-dimethyl-2-oxo-bicyclo-[2.2.1]hept-1-ylmethan Sulfonsäure) und dessen Salze (besonders die entprechenden 10-Sulfato-verbindungen, insbesondere das entsprechende Natrium-, Kalium- oder Triethanolammonium-Salz), das auch als Benzol-1,4-di(2-oxo-3-bornylidenmethyl-10-sulfonsäure) bezeichnet wird. Benzol-1,4-di(2-oxo-3-bornylidenmethyl-10-sulfonsäure) hat die INCI-Bezeichnung Terephtalidene Dicampher Sulfonsäure (CAS.-Nr.: 90457-82-2) und ist beispielsweise unter dem Handelsnamen Mexoryl SX von der Fa. Chimex erhältlich;
- Sulfonsäure-Derivate des 3-Benzylidencamphers, wie z. B. 4-(2-Oxo-3-bornylidenmethyl)-benzolsulfonsäure, 2-Methyl-5-(2-oxo-3-bornylidenmethyl)sulfonsäure und deren Salze.

Vorteilhafte UV-Filtersubstanzen im Sinne der vorliegenden Erfindung sind ferner sogenannte Breitbandfilter, d.h. Filtersubstanzen, die sowohl UV-A- als auch UV-B-Strahlung absorbieren.

Vorteilhafte Breitbandfilter oder UV-B-Filtersubstanzen sind beispielsweise Triazinderivate, wie z. B.

- 2,4-Bis-{[4-(2-Ethyl-hexyloxy)-2-hydroxy]-phenyl}-6-(4-methoxyphenyl)-1,3,5-triazin (INCI: Methylene Bis-Benzotriazolmethylbutylphenol), welches unter der Handelsbezeichnung Tinosorb® S bei der CIBA-Chemikalien GmbH

erhältlich ist;

- Dioctylbutylamidotriazon (INCI: Diethylhexylbutamidotriazone), welches unter der Handelsbezeichnung UVASORB HEB bei Sigma 3V erhältlich ist;
- 4,4',4"-(1,3,5-Triazin-2,4,6-triyltriimino)-tris-benzoësäure-tris(2-ethylhexylester), auch: 2,4,6-Tris-[anilino-(p-carbo-2'-ethyl-1'-hexyloxy)]-1,3,5-triazin (INCI: Octyl Triazone), welches von der BASF Aktiengesellschaft unter der Warenbezeichnung UVINUL® T 150 vertrieben wird.

Ein vorteilhafter Breitbandfilter im Sinne der vorliegenden Erfindung ist auch das 2,2'-Methylen-bis-(6-(2H-benzotriazol-2-yl)-4-(1,1,3,3-tetramethylbutyl)-phenol) (INCI: Bisoctyltriazol), welches unter der Handelsbezeichnung Tinosorb® M bei der CIBA-Chemikalien GmbH erhältlich ist.

Vorteilhafter Breitbandfilter im Sinne der vorliegenden Erfindung ist ferner das 2-(2H-benzotriazol-2-yl)-4-methyl-6-[2-methyl-3-[1,3,3,3-tetramethyl-1-[(trimethylsilyl)oxy]disiloxanyl]propyl]-phenol (CAS-Nr.: 155633-54-8) mit der INCI-Bezeichnung Drometrizole Trisiloxane.

Die weiteren UV-Filtersubstanzen können öllöslich sein. Vorteilhafte öllösliche Filtersubstanzen sind z. B.:

- 3-Benzylidencampher-Derivate, vorzugsweise 3-(4-Methylbenzyliden)campher, 3-Benzylidencampher;
- 4-Aminobenzoesäure-Derivate, vorzugsweise 4-(Dimethylamino)-benzoesäure(2-ethylhexyl)ester, 4-(Dimethylamino)benzoesäureamylester;
- Derivate des Benzophenons, vorzugsweise 2-Hydroxy-4-methoxybenzophenon, 2-Hydroxy-4-methoxy-4'-methylbenzophenon, 2,2'-Dihydroxy-4-methoxybenzophenon
- sowie an Polymere gebundene UV-Filter.

Eine weiterere erfindungsgemäß vorteilhaft zu verwendende Lichtschutzfiltersubstanz ist das Ethylhexyl-2-cyano-3,3-diphenylacrylat (Octocrylen), welches von BASF unter der Bezeichnung Uvinul® N 539 erhältlich ist.

Besonders vorteilhafte Zubereitungen im Sinne der vorliegenden Erfindung, enthalten neben der oder den erfindungsgemäßen Filtersubstanz(en) bevorzugt ferner weitere UV-A- und/oder Breitbandfilter, insbesondere Dibenzoylmethanderivate [beispielsweise das 4-(tert.-Butyl)-4'-methoxydibenzoylmethan] und/oder das 2,4-Bis-{[4-(2-Ethylhexyloxy)-2-hydroxy]-phenyl}-6-(4-methoxyphenyl)-1,3,5-triazin, jeweils einzeln oder in beliebigen Kombinationen miteinander.

Die Liste der genannten UV-Filter, die im Sinne der vorliegenden Erfindung eingesetzt werden können, soll selbstverständlich nicht limitierend sein.

Vorteilhaft enthält die partikuläre Phase die Substanzen, die UV-Strahlung im UV-A- und/oder UV-B-Bereich absorbieren, in einer Gesamtmenge von z. B. 0,1 Gew.-% bis 30 Gew.-%, vorzugsweise 0,5 bis 20 Gew.-%, insbesondere 1,0 bis 15,0 Gew.-%, jeweils bezogen auf das Gesamtgewicht der partikulären Phase.

Es ist möglich und erfindungsgemäß gegebenenfalls vorteilhaft, wenngleich nicht zwingend, nicht nur die partikuläre Phase sondern auch die äußere fließfähige Phase auf Gelbasis mit einem Gehalt an UV-Filtersubstanzen auszustatten, solange die Anforderungen an die Turbidität eingehalten werden. In der Regel werden in der äußeren Gelphase wasserlösliche UV-Filtersubstanzen eingesetzt.

Wenn in der äußeren Gelphase wasserlösliche UV-Filtersubstanzen eingesetzt werden sollen, enthält sie die Substanzen, die UV-Strahlung im UV-A- und/oder UV-B-Bereich absorbieren, in einer Gesamtmenge von z. B. 0,1 Gew.-% bis 30 Gew.-%, vorzugsweise 0,5 bis 20 Gew.-%, insbesondere 1,0 bis 15,0 Gew.-%, jeweils bezogen auf das Gesamtgewicht der äußeren Gelphase.

Um die Partikel herzustellen, die die innere feste partikuläre Phase konstituieren, kann vorteilhaft ein Verfahren werwendet werden, welches aus der DE 44 24 998 A1 bekannt ist. Bei diesem Verfahren wird die Zusammensetzung, aus der die partikuläre Phase besteht, bzw. ggfs. eine Zusammensetzung, aus welcher sich die partikuläre Phase entwickeln soll, und welche bevorzugt in fließfähiger Form vorliegt, zur einem der die Aushärtung bewirkenden Umgebung gerichteten Flüssigkeitsstrahls geformt wird und dass die Bildung der Portionen dadurch erfolgt, dass der Flüssigkeitsstrahl vor der die Aushärtung bewirkenden Umgebung in definierte Abschnitte so zerteilt wird, dass sich die Abschnitte im wesentlichen in Richtung des Flüssigkeitsstrahls weiterbewegen.

Dieses Verfahren kann derzeit ausgestaltet werden, dass aus der Zusammensetzung, aus der die partikuläre Phase besteht, bzw. ggfs. eine Zusammensetzung, aus welcher sich die partikuläre Phase entwickeln soll, zunächst ein Flüssigkeitsstrahl geformt wird, in dem sich die Flüssigkeitsteilchen in Richtung der die Aushärtung bewirkenden Umgebung bewegen. Nach Formung des Flüssigkeitsstrahls wird dieser in definierte Abschnitte mechanisch zerteilt, wobei die so gebildeten Abschnitte sich der durch den Flüssigkeitsstrahl vorgegebenen Richtung weiterbewegen. Die Zerteilung des Flüssigkeitsstrahls erfolgt somit durch periodisches Entfernen von Flüssigkeit aus dem Flüssigkeitsstrahl, wodurch sich die definierten Abschnitte bilden. Die Länge dieser Abschnitte kann dabei so kurz gehalten werden, dass während der verbleibenden Bewegung bis zur die Aushärtung bewirkenden Umgebung, insbesondere die Oberfläche eines Härtungsmediums, aufgrund der Oberflächenspannung des flüssigen Mediums eine zumindest angenäherte Kugelform ausgebildet wird, damit kugelförmige Teilchen entstehen.

Bei flüssigen Medien, die eine gewisse Viskosität aufweisen, ist es zweckmäßig, wenn das flüssige Medium zur Ausbildung des Flüssigkeitsstrahls durch die Düse gedrückt wird. Dies geschieht üblicherweise durch die Anwendung eines Überdrucks im Vorratsbehälter bzw. in der Leitung zur Flüssigkeitsstrahldüse.

In vielen Anwendungsfällen besteht das Bedürfnis, das vorzugsweise kugelförmige Teilchen mit einer Beschichtung zu versehen. diese Beschichtung gegebenenfalls in derselben Umgebung aushärten kann, ist es gemäß der vorliegenden Erfindung möglich, die gebildeten Abschnitte des flüssigen Mediums vor der Aushärtung bereits zu beschichten, indem wenigstens ein Flüssigkeitsquerstrahl mit einer zur Umhüllung geeigneten Flüssigkeit erzeugt wird, durch den die gebildeten Abschnitte hindurchtreten.

Der Flüssigkeitsquerstrahl kann dabei zweckmäßigerweise als Flachstrahl ausgebildet sein, so dass die mögliche Durchtrittsfläche für die Abschnitte vergrößert wird.

Eine Modifikation des Verfahrens kann darin bestehen, dass die Düsenanordnung zur Ausbildung wenigstens eines Flüssigkeitsstrahls eingerichtet ist und unterhalb der Düsenanordnung eine bewegbare Teilvorrichtung für den wenigstens einen Flüssigkeitsstrahl angeordnet ist.

Die Teilvorrichtung kann dabei unmittelbar am Ausgang der Düsenanordnung positioniert sein.

Vorzugsweise ist die bewegte Teilvorrichtung rotierend antreibbar und mit den Flüssigkeitsstrahl unterbrechenden Elementen ausgestattet. Durch den rotierenden Antrieb der Teilvorrichtung läßt sich ein besonders einfacher Aufbau der erfindungsgemäßen Vorrichtung erreichen.

Die den Flüssigkeitsstrahl unterbrechenden Elemente können in einer vorteilhaften Ausführungsform radial ausgerichtete strahlenförmige Elemente sein, die durch Drähte, Kunststoffäden o. ä. realisiert sind. Die Elemente können dabei ohne eigene Formstabilität ausgebildet sein und ihre radiale Ausrichtung erst während der Rotation der Teilvorrichtung erhalten.

In einer anderen Ausführungsform kann eine rotierende Scheibe mit auf einem Radius dicht nebeneinander angeordneten Durchgangsöffnungen für den Flüssigkeitsstrahl verwendet werden. Die Stege zwischen den Durchgangsöffnungen bilden dabei die den Flüssigkeitsstrahl unterbrechenden Elemente.

Zweckmäßigerweise ist die Teilvorrichtung von einem zylindrischen, wenigstens im Bereich des Flüssigkeitsstrahls offenen Gehäuse umgeben, das eine Auffangvertiefung zum Auffangen von an der Gehäusewandung herablaufender Flüssigkeit aufweist. Auf diese Weise wird die durch die den Flüssigkeitsstrahl unterbrechenden Elemente nach radial außen geschleuderte Flüssigkeit aufgefangen und kann dem zugehörigen Vorratsbehälter wieder zugeführt werden. Die Auffangvertiefung ist dabei vorzugsweise durch eine umlaufende Rille gebildet, die mit einem Abfluß versehen ist.

Zur Ausbildung einer obenerwähnten Beschichtung der Teilchen kann die Vorrichtung eine bezüglich des Flüssigkeitsstrahls stromabwärts von der Teilvorrichtung angeordnete, im Winkel zum Flüssigkeitsstrahl gerichtete Querdüse aufweisen, die mit einem Vorratsbehälter für eine zur Umhüllung geeignete Flüssigkeit verbunden ist. Die Querdüse kann dabei eine Flachstrahldüse darstellen.

In einer beispielhaften Ausführungsform kann aus der Düse ein Flüssigkeitsstrahl einer Zellenemulsion in einer Natrium-Alginatlösung austreten und der Querstrahl durch eine zellenfreie Natrium-Alginatlösung gebildet sein, während sich im Auffangbehälter 5 eine $CaCl_2$-Lösung als Härtungsmedium befindet.

Beim Durchtritt durch den vorzugsweise als Flachstrahl ausgebildeten Querstrahl werden die zellenhaltigen Flüssigkeitsabschnitte 3 min mit der zellenfreien Alginatlösung umhüllt und anschließend sofort vernetzt, so dass eine Wanderung der Zellen aus dem Kern in die Umhüllungsschicht nicht eintreten kann, da die Ca-Ionen wesentlich schneller in den Alginattropfen diffundieren als sich die Zellen bewegen können.

Es ist möglich, einen üblichen kosmetischen Behälter mit einer erfindungsgemäßen Zubereitung so auszustatten, dass die innere partikuläre Phase an eine dichtestmögliche Kugelpackung heranreicht (Raumsausfüllung der kubisch dichtesten Kugelpackung: etwa 74 %), deren Hohlräume durch die äußere wäßrige Gelphase ausgefüllt werden.

Dies ist jedoch nicht immer erwünscht und hat auch nicht immer praktische Vorteile. Bevorzugt ist daher, dass das Volumenverhältnis von innerer partikulärer Phase zu äußerem wäßrgen Gel aus dem Bereich von etwa 20% : 80% bis 50% : 50% gewählt werden.

Optisch ansprechend, vorteilhaft und in diesem weiteren Aspekt eigenständig erfinderisch können erfindungsgemäße Zubereitungen in einem

einen Behälter (B),

- der einen röhrenförmigen Korpus (K),
  - dessen Wandung (Wk) eine Transmission für sichtbares Licht von 25 - etwa 100 % aufweist,
- auf dessen oberem Ende ein Spenderkopf (SK) angebracht ist,
- der unten von einem beweglichen Schleppkolben (SCH) begrenzt ist,

vorgehalten werden.

Besonders vorteilhaft kann der Spenderkopf (SK)

- ein äußeres Betätigungselement (BE) mit einer Spendeöffnung (SPO), welche durch ein Rückschlagventil (RV) verschlossen ist,
- eine auf dem röhrenförmigen Korpus (K) aufliegenden Bodeneinheit (BO) mit einer Öffnung (O) zum röhrenförmigen Korpus, sowie
- einen flexiblen inneren im wesentlich zylindrisch ausgeformten Hohlkörper (H) mit balgenartigen Wandungen (Wh) umfassen,

  wobei die Elemente des Spenderkopfes so ausgestaltet sind, dass

  - der Hohlkörper (H) an den beiden Stirnenden (So), (Su) jeweils Öffnungen (Oo), (Ou) aufweist,
  - der Hohlkörper (H) mit dem unteren Stirnende (Su) auf der Bodeneinheit (BO) aufliegt, und wobei deren Öffnung (O) und die untere Öffnung (Ou) des Hohlkörpers (H) übereinander liegen,

    sodass, wenn
  - das Betätigungselement (BE) durch äußeren Druck den inneren Hohlkörper (H) in Richtung dessen Längsachse zusammendrückt.

- die sich im Innern des Hohlkörpers (H) befindliche Substanz durch das Rückschlagventil (RV und den Spenderkopf (SK) hindurch aus dem Behälter (B) herausbefördert wird, und
- bei Nachlassen des äußeren Druckes die Rückstellkraft des flexiblen inneren Hohlkörpers (H) Substanz aus dem röhrenförmigen Korpus (K in den flexiblen inneren Hohlkörper (H) gesogen wird.

Fig. 1 zeigt einen erfindungsgemäß vorteilhaften Behälter (B),

- der einen röhrenförmigen Korpus (K),
- auf dessen oberem Ende ein Spenderkopf (SK) angebracht ist,
- der unten von einem beweglichen Schleppkolben (SCH) begrenzt ist, und
- einen Spenderkopf (SK) aufweist,

wobei in Fig. 1a der Behälter (B) in Korpus (K) und Spenderkopf (SK) (hier mit einer zusätzlichen, nicht näher bezeichneten Verschlußkappe versehen) aufgetrennt dargestellt ist.

Fig. 1 a zeigt einen Spenderkopf (SK),

- der ein äußeres Betätigungselement (BE) mit einer Spendeöffnung (SPO), welche durch ein Rückschlagventil (RV) verschlossen ist,
- eine auf dem röhrenförmigen Korpus (K) aufliegenden Bodeneinheit (BO) mit einer Öffnung (O) zum röhrenförmigen Korpus, sowie
- einen flexiblen inneren im wesentlich zylindrisch ausgeformten Hohlkörper (H) mit balgenartigen Wandungen (Wh) umfaßt.

Fig. 1b zeigt eine perspektivische Ansicht einer Ausführungsform des flexiblen inneren im wesentlich zylindrisch ausgeformten Hohlkörpers (H)

- mit balgenartigen Wandungen (Wh),
- wobei das untere Stirnende (Su) und deren Öffnung (Ou) sichtbar ist.

Fig. 1c zeigt den Aufriß einer Ausführungsform des flexiblen inneren im wesentlich zylindrisch ausgeformten Hohlkörpers (H)

- mit balgenartigen Wandungen (Wh),

  wobei die Elemente des Spenderkopfes so ausgestaltet sind, dass
- der Hohlkörper (H) an den beiden Stirnenden (So), (Su) jeweils Öffnungen (Oo), (Ou) aufweist,

und wobei der Hohlkörper (H) so ausgestaltet ist, dass die untere Öffnung (Ou) um unteren Stirnende (Su) eine Vorrichtung (V) umfaßt, die den Substanzstrom aus dem Innern des röhrenförmigen Korpus (K) in den Hohlkörper hinein in mehrere Teilströme zerschneidet.

Fig. 1d zeigt eine Vorrichtung (V), die dafür vorgesehen ist, den Substanzstrom aus dem Innern des röhrenförmigen Korpus (K) (hier nicht aufgeführt) in den Hohlkörper (H) (hier nicht aufgeführt) hinein mittels dreier zwei Durchlaßöffnungen (Du) in drei Teilströme zerschneiden soll.

In den erfindungsgemäßen Zubereitungen sind das oder die Ubichinone und/oder Plastochinone bestens gegen Oxidation und schädlichen Einfluß von UV-Strahlung geschützt.

Beim Auftragen der Zubereitungen auf eine Oberfläche lassen sich die erfindungsgemäßen Zubereitungen zu einer homogenen Emulsion verreiben, die schnell in die Haut einzieht und optisch wie haptisch äußerst ansprechend ist.

Besonders überraschend ist, dass, wenn ein Spender verwendet wird, die Scherkräfte am Spenderausgang bereits genügen, um die ursprünglich in getrennten Kompartimenten (also äußere Gelphase und innere partikuläre Phase) zu einer optisch homogenen Einheit zu verwandeln.

Die kosmetischen und dermatologischen Zubereitungen gemäß der Erfindung können kosmetische Hilfsstoffe enthalten, wie sie üblicherweise in solchen Zubereitungen verwendet werden, z. B. Konservierungsmittel, Konservierungshelfer, Bakterizide, Parfüme, Substanzen zum Verhindern des Schäumens, Farbstoffe, Pigmente, die eine färbende Wirkung haben, Verdickungsmittel, anfeuchtende und/oder feuchhaltende Substanzen, Füllstoffe, die das Hautgefühl verbessern, Fette, Öle, Wachse oder andere übliche Bestandteile einer kosmetischen oder dermatologischen Formulierung wie Alkohole, Polyole, Polymere, Schaumstabilisatoren, Elektrolyte, organische Lösungsmittel oder Silikonderivate.

Vorteilhafte Konservierungsmittel im Sinne der vorliegenden Erfindung sind beispielsweise Formaldehydabspalter (wie z. B. DMDM Hydantoin, welches beispielsweise unter der Handelsbezeichnung Glydant ™ von der Fa. Lonza erhältlich ist), Iodopropylbutylcarbamate (z. B. die unter den Handelsbezeichnungen Glycacil-L, Glycacil-S von der Fa. Lonza erhältlichen und/oder Dekaben LMB von Jan Dekker), Parabene (d. h. p-Hydroxybenzoesäurealkylester, wie Methyl-, Ethyl-, Propyl- und/oder Butylparaben), Phenoxyethanol, Ethanol, Benzoesäure und dergleichen mehr. Üblicherweise umfaßt das Konservierungssystem erfindungsgemäß ferner vorteilhaft auch Konservierungshelfer, wie beispielsweise Octoxyglycerin, Glycine Soja etc.

Besonders vorteilhafte Zubereitungen werden ferner erhalten, wenn als Zusatz- oder Wirkstoffe Antioxidantien eingesetzt werden. Erfindungsgemäß enthalten die Zubereitungen vorteilhaft eines oder mehrere Antioxidantien. Als günstige, aber dennoch fakultativ zu verwendende Antioxidantien können alle für kosmetische und/oder dermatologische Anwendungen geeigneten oder gebräuchlichen Antioxidantien verwendet werden.

Besonders vorteilhaft im Sinne der vorliegenden Erfindung können wasserlösliche Antioxidantien eingesetzt werden, wie beispielsweise Vitamine, z. B. Ascorbinsäure und deren Derivate sowie D-Biotin, natürliche und/oder synthetische Isoflavonoide, alpha-Glucosylrutin, Panthenol, Aloe Vera, Honokiol, Magnolol.

Die Menge der Antioxidantien (eine oder mehrere Verbindungen) in den Zubereitungen beträgt vorzugsweise 0,001 bis 30 Gew.-%, besonders bevorzugt 0,05 bis 20 Gew.-%, insbesondere 0,1 bis 10 Gew.-%, bezogen auf das Gesamtgewicht der Zubereitung.

Es ist insbesondere vorteilhaft, wenn die kosmetischen Zubereitungen gemäß der vorliegenden Erfindung kosmetische oder dermatologische Wirkstoffe enthalten, wobei bevorzugte Wirkstoffe Antioxidantien sind, welche die Haut vor oxidativer Beanspruchung schützen können.

Vorteilhafte weitere Wirkstoffe sind natürliche Wirkstoffe und/oder deren Derivate, wie z. B. Phytoen, Carnitin, Carnosin, Kreatin, N-Acetyl-Hydroxyprolin, Taurin und/oder β-Alanin.

Die äußere wäßrige Gelphase der erfindungsgemäßen Zubereitungen kann vorteilhaft übliche kosmetische Hilfsstoffe enthalten, wie beispielsweise Alkohole, insbesondere solche niedriger C-Zahl, vorzugsweise Ethanol und/oder Isopropanol, Diole oder Polyole niedriger C-Zahl sowie deren Ether, vorzugsweise Propylenglykol, Glycerin, Ethylenglykol, Ethylenglykolmonoethyl- oder -monobutylether, Propylenglykolmonomethyl, -monoethyl- oder -monobutylether, Diethylenglykolmonomethyl- oder -monoethylether und analoge Produkte, Polymere, Schaumstabilisatoren, Elektrolyte sowie insbesondere ein oder mehrere Verdickungsmittel, welches oder welche vorteilhaft gewählt werden können aus der Gruppe Siliciumdioxid, Aluminiumsilikate, Polysaccharide bzw. deren Derivate, z. B. Hyaluronsäure, Xanthangummi, Hydroxypropylmethylcellulose, besonders vorteilhaft aus der Gruppe der Polyacrylate, bevorzugt ein Polyacrylat aus der Gruppe der sogenannten Carbopole, beispielsweise Carbopole der Typen 980, 981, 1382, 2984, 5984, jeweils einzeln oder in Kombination. Auch Moisturizer können bevorzugt verwendet werden.

Als Moisturizer werden Stoffe oder Stoffgemische bezeichnet, welche kosmetischen oder dermatologischen Zubereitungen die Eigenschaft verleihen, nach dem Auftragen bzw. Verteilen auf der Hautoberfläche die Feuchtigkeitsabgabe der Hornschicht (auch transepidermal water loss (TEWL) genannt) zu reduzieren und/oder die Hydratation der Hornschicht positiv zu beeinflussen.

Vorteilhafte Moisturizer im Sinne der vorliegenden Erfindung sind beispielsweise Glycerin, Milchsäure und/oder Lactate, insbesondere Natriumlactat, Butylenglykol, Propylenglykol, Biosaccaride Gum-1, Glycine Soja, Ethylhexyloxyglycerin, Pyrrolidoncarbonsäure und Harnstoff. Ferner ist es insbesondere von Vorteil, polymere Moisturizer aus der Gruppe der wasserlöslichen und/oder in Wasser quellbaren und/oder mit Hilfe von Wasser gelierbaren Polysaccharide

zu verwenden. Insbesondere vorteilhaft sind beispielsweise Hyaluronsäure, Chitosan und/oder ein fucosereiches Polysaccharid, welches in den Chemical Abstracts unter der Registraturnummer 178463-23-5 abgelegt und z. B. unter der Bezeichnung Fucogel®1000 von der Gesellschaft SOLABIA S.A. erhältlich ist.

Die erfindungsgemäßen kosmetischen oder dermatologischen Zubereitungen können ferner vorteilhaft, wenngleich nicht zwingend, Füllstoffe enthalten, welche z. B. die sensorischen und kosmetischen Eigenschaften der Formulierungen weiter verbessern und beispielsweise ein samtiges oder seidiges Hautgefühl hervorrufen oder verstärken. Vorteilhafte Füllstoffe im Sinne der vorliegenden Erfindung sind Stärke und Stärkederivate (wie z. B. Tapiocastärke, Distärkephosphat, Aluminium- bzw. Natrium-Stärke Octenylsuccinat und dergleichen), Pigmente, die weder hauptsächlich UV-Filter- noch färbende Wirkung haben (wie z. B. Bornitrid etc.) und/oder Aerosile® (CAS-Nr. 7631-86-9).

Die nachfolgenden Beispiele sollen die vorliegende Erfindung verdeutlichen, ohne sie einzuschränken. Die Zahlenwerte in den Beispielen bedeuten Gewichtsprozente, bezogen auf das Gesamtgewicht der jeweiligen Zubereitungen.

Beispiele innere partikuläre Phase:

| | **Varianten** | | | | |
|---|---|---|---|---|---|
| **INCI** | **A** | **B** | **C** | **D** | **E** |
| Algin | 1,5 | 1,5 | 1,5 | 1,5 | 1,5 |
| Ubichinon | 0,19 | 0,1 | 0,25 | 0,13 | 0,2 |
| CI 77891 | 2,50 | 1 | 0,5 | 2 | 0,3 |
| Cyclomethicon (BRB CM 56; BRB International) | 10,00 | 15 | 5 | 8 | 12 |
| Cyclomethicon + Dimethiconol (Xiameter PMX 1501 Fluid; Dow Corning) | 10,00 | 5 | 15 | 12 | 8 |
| Natriumstearylglutamat (Eumulgin SG; BASF Personal Care and Nutrition) | 0,25 | 1,1 | - | - | - |
| Polyglyceryl-3 Methylglucose Distearat (Tego Care 450; Evonik Industries) | - | - | 0,5 | 1 | 2 |
| Polymethylsilsesquioxane (Gransil PSQ; Grant Industries) | 8,00 | 4 | 10 | 8 | 12 |
| Parfum | 1,00 | 0,49 | 0,6 | 0,8 | 0,4 |
| Phenoxyethanol | 0,60 | 0,6 | 0,6 | 0,6 | 0,6 |
| 1,2-Hexandiol | 0,50 | 0,5 | 0,5 | 0,5 | 0,5 |
| Methylpropandiol | 2,00 | 2 | 2 | 2 | 2 |
| Wasser | ad 100 | ad 100 | ad 100 | ad 100 | ad 100 |

Die Rezepturbestandteile der Beispiele A bis E werden analog zum Versuchsbeispiel in der DE 44 24 998, Spalte 5, bearbeitet und zu annähernd spharischen Partikeln des Durchmessers ca 6mm ausgeformt

| Beispiele äußeres wässriges Gel: | **Varianten** | | | | | | | |
|---|---|---|---|---|---|---|---|---|
| **INCI** | **1** | **2** | **3** | **4** | **5** | **6** | **7** | **8** |
| Kreatin | 0,04 | 0,07 | 0,04 | 0,07 | 0,04 | 0,07 | 0,04 | 0,07 |
| Kreatinin | 0,01 | 0,02 | 0,01 | 0,02 | 0,01 | 0,02 | 0,01 | 0,02 |
| Natriumhyaluronat | 0,2 | 0,4 | 0,2 | 0,4 | 0,2 | 0,4 | 0,2 | 0,4 |
| PEG-40 hydriertes Rizinusöl (Eumulgin CO 40; BASF Personal Care and Nutrition) | 0,5 | 0,8 | 0,5 | 0,8 | 0,5 | 0,8 | 0,5 | 0,8 |

(fortgesetzt)

| Beispiele äußeres wässriges Gel: | **Varianten** | | | | | | | |
|---|---|---|---|---|---|---|---|---|
| **INCI** | **1** | **2** | **3** | **4** | **5** | **6** | **7** | **8** |
| Glycerin | 15 | 22 | 15 | 22 | 15 | 22 | 15 | 22 |
| 1,2-Propandiol | 4 | 5 | 4 | 5 | 4 | 5 | 4 | 5 |
| Natriumcitrat (Tri-Natriumcitrat-5,5-hydrat reinst Nr. 106431; Merck) | 1,05 | 1,63 | 1,05 | 1,63 | 1,05 | 1,63 | 1,05 | 1,63 |
| Natriumhydroxid 45% | 0,8 | 1 | 0,8 | 1 | 0,8 | 1 | 0,8 | 1 |
| Phenoxyethanol | 0,6 | 0,6 | 0,6 | 0,6 | 0,6 | 0,6 | 0,6 | 0,6 |
| 1,2-Hexandiol | 0,5 | 0,5 | 0,5 | 0,5 | 0,5 | 0,5 | 0,5 | 0,5 |
| Methylpropandiol | 2 | 2 | 2 | 2 | 2 | 2 | 2 | 2 |
| Acrylsäure/VP Crosspolymer (Ultrathix P-100; Ashland Specialty Ingredients) | 1,28 | 2,33 | - | - | - | - | - | - |
| Acrylates/C10-30 Alkylacrylat Crosspolymer | - | - | 0,7 | 1,63 | - | - | - | - |
| Ammonium Acryloyldimethyltaurate/VP Copolymer (Aristoflex AVC; Clariant) | - | - | - | - | 2 | 2,5 | - | - |
| Carbomer (Carbopol 980 or Carbopol 981; Lubrizol Advanced Materials) | - | - | - | - | - | - | 0,7 | 1,63 |
| Lubrajel Oil Free; Ashland Specialty Ingredients | 4 | 5 | 4 | 5 | 4 | 5 | 4 | 5 |
| Wasser | ad 100 | ad 100 | ad 100 | ad 100 | ad 100 | ad 100 | ad 100 | ad 100 |

Die Rezepturbestandteile der Beispiele 1 - 8 werden bei 25 °C miteinander verrührt, bis sich klare Gele gebildet haben.

Partikel und Gele wurden in verschiedenen Volumenverhältnissen miteinander kombiniert, wie in folgenden Kombinierten Beispielen angeführt. Der Erste Wert gibt die partikuläre Phase, der zweite Wert die Gelphase wieder

| | 1 | 2 | 3 | 4 | 5 | 6 | 7 | 8 |
|---|---|---|---|---|---|---|---|---|
| *A* | 25 : 75 | 30 : 70 | 50 : 50 | 40 : 60 | 25 : 75 | 40 : 60 | 40 : 60 | 50 : 50 |
| | | | | | | | | |
| *B* | 30 : 70 | 40 : 60 | 25 : 75 | 30 : 70 | 40 : 60 | 35 : 65 | 35 : 65 | 25 : 75 |
| | | | | | | | | |
| *C* | 50 : 50 | 40 : 60 | 40 : 60 | 50 : 50 | 35 : 65 | 25 : 75 | 50 : 50 | 40 : 60 |
| | | | | | | | | |
| *D* | 40 : 60 | 35 : 65 | 35 : 65 | 35 : 65 | 30 : 70 | 40 : 60 | 30 : 70 | 50 : 50 |
| | | | | | | | | |
| *E* | 25 : 75 | 35 : 65 | 50 : 50 | 35 : 65 | 40 : 60 | 35 : 65 | 40 : 60 | 35 : 65 |
| Je 40 ml der Zubereitungen A1 - E 8 werde,n jede für sich, in Behälter, wie in den Figuren Fig.1a bis Fig 1d beschrieben, abgefüllt. → | | | | | | | | |

**Patentansprüche**

1. Kosmetische Zubereitungen, umfassend

   - eine äußere fließfähige gelartige Phase auf wäßriger Basis

     - welche eine Transmission für Licht der Wellenlänge von 700 nm aus dem Bereich von 30 bis 100 % aufweist,
     - welche eine Viskosität gewählt aus dem Intervall von 1000 - 10000 mPas bei einer Temperatur von 25 °C und einer Scherrate von ca. 10, vorzugsweise von von 3000 - 9000 bei einer Temperatur von 25 °C und einer Scherrate von ca. 10, insbesondere bevorzugt von 5000 -8000 bei einer Temperatur von 25 °C und einer Scherrate von ca. 10 aufweist,
     und
     - welche gegebenenfalls übliche, in Wasser lösliche oder dispergierbare Hilfs- und oder Zusatzstoffe enthält,

   - eine innere feste partikuläre Phase bestehend im wesentlichen aus kugelförmigen Partikel, deren mittlerer Durchmesser gewählt wird aus dem Bereich von 0,1 bis 10 mm, bevorzugt 2 bis 7,5 mm, und welche umfassen:

     - eine Polymermatrix, welche im Wesentlichen unlöslich in Wasser ist,
     - eine oder mehrere Substanzen gewählt aus der Gruppe der Ubichinone und/oder Plastochinone
     - eine oder mehrere Lichtfiltersubstanzen, welche gewählt wird oder werden aus der Gruppe der Metalloxidpigmente,
     - gegebenenfalls eine oder mehrere Lichtfiltersubstanzen, welche gewählt wird oder werden aus der Gruppe der üblichen kosmetischen Lichtschutzfiltersubstanzen.

2. Zubereitungen nach Anspruch 1, **dadurch gekennzeichnet, dass** das oder die Hydrokolloide gewählt wird oder werden aus der Gruppe der Cellulosen/Methylcellulosen, der Polyacrylate und besonders bevorzugt aus der Gruppe des Carrageens, des Xanthans, des "Acrylates/C 10-30 Alkyl Acrylate Crosspolymers" und der Ammoniumacryloyldimethyltaurate/Vinylpyrrolidoncopolymere.

3. Zubereitungen nach einem der vorstehenden Ansprüche, **dadurch gekennzeichnet, dass** die Gesamtmenge an einem oder mehreren Hydrokolloiden vorteilhaft kleiner als 1,5 Gew.-%, bevorzugt zwischen 0,1 und 1,0 Gew.-%, bezogen auf das Gesamtgewicht der Zubereitungen, gewählt wird.

4. Partikel oder Zubereitungen nach einem der vorstehenden Ansprüche, **dadurch gekennzeichnet, dass** die Polymermatrix der inneren partikulären Phase gewählt wird aus der Gruppe folgender Polymermatrices: Algin/Alginat, Carrageen, Agar, Gellan Gum, Chitosan.

5. Partikel oder Zubereitungen nach einem der vorstehenden Ansprüche, **dadurch gekennzeichnet, dass** das oder die Metalloxidpigmente gewählt wird oder werden aus der Gruppe der Oxide des Titans ($TiO_2$), Zinks (ZnO), Eisens (z. B. $Fe_2O_3$), Zirkoniums ($ZrO_2$), Siliciums ($SiO_2$), Mangans (z. B. MnO), Aluminiums ($Al_2O_3$), Cers (z. B. $Ce_2O_3$), Mischoxide der entsprechenden Metalle sowie Abmischungen aus solchen Oxiden sowie das Sulfat des Bariums ($BaSO_4$).

6. Partikel oder Zubereitungen nach einem der vorstehenden Ansprüche, **dadurch gekennzeichnet, dass** das oder die Metalloxidpigmente gewählt wird oder werden aus der Gruppe der Oxide des Titans ($TiO_2$).

7. Partikel oder Zubereitungen nach einem der vorstehenden Ansprüche, **dadurch gekennzeichnet, dass** als Ubichinon das Ubichinon 10 (Coenzxym Q10) gewählt wird. Reduzierte Form: Ubichinol.

8. Partikel oder Zubereitungen nach einem der vorstehenden Ansprüche, **dadurch gekennzeichnet, dass** die Lichtschutzfiltersubstanz oder -Substanzen in der partikulären Phase gewählt wird oder wernden aus der Gruppe Octylmethoxycinnamate, Octocrylene, Tinosorb M, Tinosorb S, Mexoryl XL, Benzophenone-3, Benzophenone-4.

9. Zubereitungen nach einem der vorstehenden Ansprüche, **dadurch gekennzeichnet, dass** die Volumenverhältnis von innerer partikulärer Phase zu äußerem wässrigen Gel aus dem Bereich von etwa 20% : 80% bis 74% : 26% gewählt werden, besonders bevorzugt 30% : 70% bis 65% zu 35 %, insbesondere bevorzugt von 40% zu 60% bis 60% : 40%.

## Claims

1. Cosmetic preparations, comprising

   - an outer flowing water-based gel-like phase

     - that features a transmission for light having a wavelength of 700 nm within the range of 30 to 100%,
     - which has a viscosity selected from the range of 1000 - 10000 mPas at a temperature of 25°C and a shear rate of approx. 10, preferably, of 3000 - 9000 at a temperature of 25°C and a shear rate of approx. 10, particularly preferred of 5000 - 8000 at a temperature of 25°C and a shear rate of approx. 10, and
     - which, if applicable, contains common excipients or additives that are water-soluble or dispersible,

   - an inner solid particulate phase essentially consisting of spherical particles, the average diameter of which is selected from a range of 0.1 to 10 mm, preferably 2 to 7.5 mm and which comprise:
   - a polymer matrix, which is essentially insoluble in water,
   - one or a plurality of substances selected from the group of ubiquinones and/or plastoquinones,
   - one or a plurality of light filter substances, which is/are selected from the group of metal oxide pigments,
   - if applicable, one or a plurality of light-filter substances, which is/are selected from the group of common cosmetic light-protection-filter substances.

2. Preparations according to Claim 1, **characterized in that** the hydrocolloid(s) is/are selected from the group of celluloses/methyl celluloses, polyacrylates and, being particularly preferred, from the group of carrageenans, xanthans, "acrylates/C 10-30 alkyl acrylates cross-polymers" and ammonium acryloyldimethyltaurates/vinylpyrrolidone-copolymers.

3. Preparations according to any one of the preceding claims, **characterized in that** the entire amount of one or a plurality of hydrocolloids is favourably selected to be less than 1.5% by weight, preferably between 0.1 and 1.0% by weight with reference to the total weight of the preparations.

4. Particles or preparations according to any one of the preceding claims, **characterized in that** the polymer matrix of the inner particulate phase is selected from the group of the following polymer matrices: Algin/alginate, carrageenan, agar, gellan gum, chitosan.

5. Particles or preparations according to any one of the preceding claims, **characterized in that** the metal-oxide pigment(s) is/are selected from the group of oxides of titanium ($TiO_2$), zinc ($ZnO$), iron (e.g. $Fe_2O_3$), zirconium ($ZrO_2$), silicon ($SiO_2$), manganese (e.g. $MnO$), aluminium ($Al_2O_3$), cerium (e.g. $Ce_2O_3$), mixed oxides of the corresponding metals and mixtures of such oxides as well as barium sulphate ($BaSO_4$).

6. Particles or preparations according to any one of the preceding claims, **characterized in that** the metal-oxide pigment(s) is/are selected from the group of titanium oxides ($TiO_2$).

7. Particles or preparations according to any one of the preceding claims, **characterized in that** ubiquinone 10 (coenzyme Q10) is selected as ubiquinone. Reduced form: Ubiquinol.

8. Preparations according to any one of the preceding claims, **characterized in that** the light-protection-filter substance or substances in the particulate phase is/are selected from the group of octyl methoxycinnamates, octocrylenes, Tinsorb M, Tinsorb S, Mexoryl XL, benzophenone-3, benzophenone-4.

9. Preparations according to any one of the preceding claims, **characterized in that** the volume ratio of the inner particulate phase to the outer aqueous gel is selected from a range of approximately 20% : 80% up to 74% : 26%, particularly preferred 30% : 70% up to 65% to 35%, especially preferred 40% to 60% up to 60% : 40%.

## Revendications

1. Préparations cosmétiques, comprenant

- une phase de type gel fluide externe à base aqueuse

  - qui présente une transmission pour la lumière de longueur d'onde de 700 nm dans la plage de 30 à 100 %,
  - qui présente une viscosité choisie dans l'intervalle de 1 000 - 10 000 mPa.s à une température de 25 °C et à une vitesse de cisaillement d'environ 10, de préférence de 3 000 - 9 000 à une température de 25 °C et à une vitesse de cisaillement d'environ 10, de façon particulièrement préférée de 5 000 - 8 000 à une température de 25 °C et à une vitesse de cisaillement d'environ 10,
  et
  - qui contient éventuellement des adjuvants ou additifs usuels, solubles ou dispersables dans l'eau,

- une phase particulaire solide interne consistant essentiellement en des particules sphériques dont le diamètre moyen est choisi dans la plage de 0,1 à 10 mm, de préférence de 2 à 7,5 mm, et qui comprennent :
- une matrice polymère qui est essentiellement insoluble dans l'eau,
- une ou plusieurs substances choisies dans le groupe des ubiquinones et/ou plastoquinones
- une ou plusieurs substance(s) filtrant la lumière, qui est ou sont choisie(s) dans le groupe des pigments de type oxyde métallique,
- éventuellement une ou plusieurs substance(s) filtrant la lumière, qui est ou sont choisie(s) dans le groupe des substances filtrantes photoprotectrices cosmétiques usuelles.

**2.** Préparations selon la revendication 1, **caractérisée en ce que** l'hydrocolloïde ou les hydrocolloïdes est ou sont choisi(s) dans le groupe des celluloses/méthylcelluloses, des polyacrylates et de façon particulièrement préférée dans le groupe du carraghénane, du xanthane, de l'« acrylates/C 10-30 alkyl acrylate crosspolymer » et des copolymères ammonium acryloyldiméthyltaurate/vinylpyrrolidone.

**3.** Préparations selon l'une quelconque des revendications précédentes, **caractérisées en ce que** la quantité totale d'un ou de plusieurs hydrocolloïdes est choisie avantageusement inférieure à 1,5 % en poids, de préférence entre 0,1 et 1,0 % en poids, par rapport au poids total des préparations.

**4.** Particules ou préparations selon l'une quelconque des revendications précédentes, **caractérisées en ce que** la matrice polymère de la phase particulaire interne est choisie dans le groupe des matrices polymères suivantes : algine/alginate, carraghénane, agar, gomme gellane, chitosane.

**5.** Particules ou préparations selon l'une quelconque des revendications précédentes, **caractérisées en ce que** le ou les pigment(s) de type oxyde métallique est ou sont choisi(s) dans le groupe des oxydes du titane ($TiO_2$), du zinc (ZnO), du fer (par exemple $Fe_2O_3$), du zirconium ($ZrO_2$), du silicium ($SiO_2$), du manganèse (par exemple MnO), de l'aluminium ($Al_2O_3$), du cérium (par exemple $Ce_2O_3$), des oxydes mixtes des métaux correspondants ainsi que des mélanges de tels oxydes ainsi que le sulfate du baryum ($BaSO_4$).

**6.** Particules ou préparations selon l'une quelconque des revendications précédentes, **caractérisées en ce que** le ou les pigment(s) de type oxyde métallique est ou sont choisi(s) dans le groupe des oxydes du titane ($TiO_2$).

**7.** Particules ou préparations selon l'une quelconque des revendications précédentes, **caractérisées en ce qu'**en tant qu'ubiquinone on choisit l'ubiquinone 10 (coenzyme Q10). Forme réduite : ubiquinol.

**8.** Particules ou préparations selon l'une quelconque des revendications précédentes, **caractérisées en ce que** la substance ou les substances filtrante(s) photoprotectrice(s) dans la phase particulaire est ou sont choisie(s) dans le groupe constitué par les octyl-méthoxycinnamates, les octocrylènes, le Tinosorb M, le Tinosorb S, le Mexoryl XL, la benzophénone-3, la benzophénone-4.

**9.** Préparations selon l'une quelconque des revendications précédentes, **caractérisées en ce que** le rapport volumique de la phase particulaire interne au gel aqueux externe est choisi dans la plage d'environ 20 % : 80 % à 74 % : 26 %, de façon particulièrement préférée de 30 % : 70 % à 65 % : 35 %, de façon plus particulièrement préférée de 40 % : 60 % à 60 % : 40 %.

Fig. 1 a

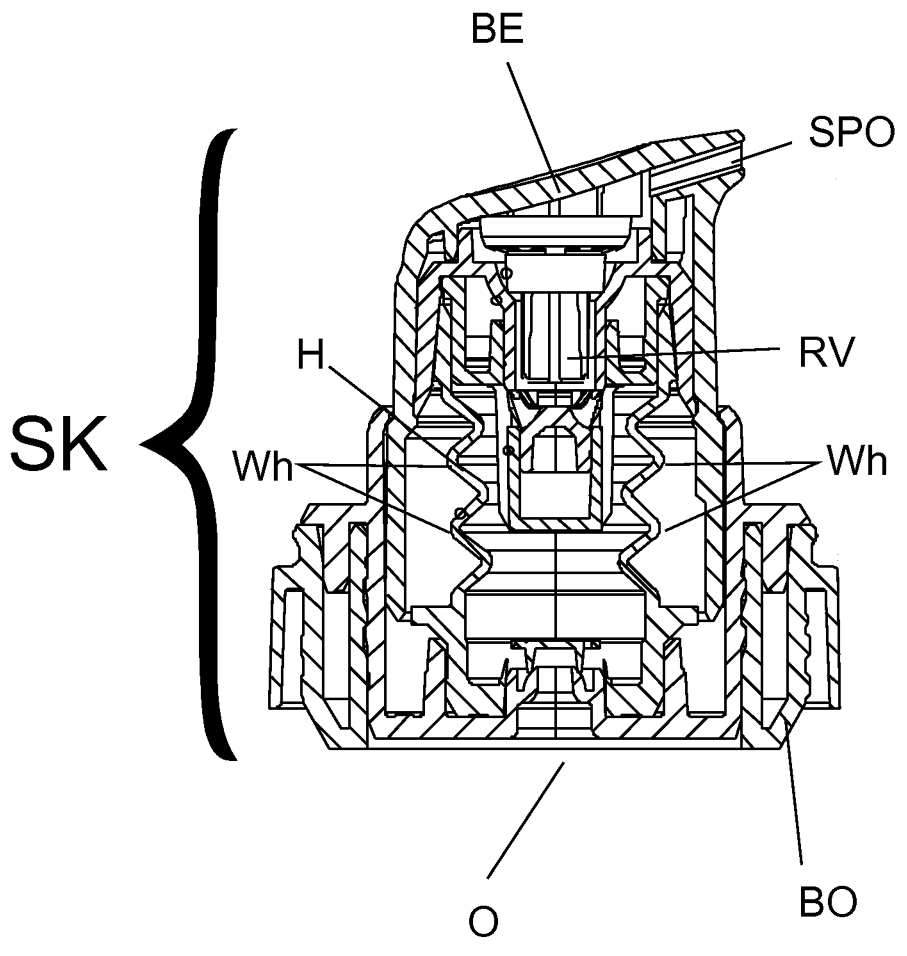

Fig. 1 b

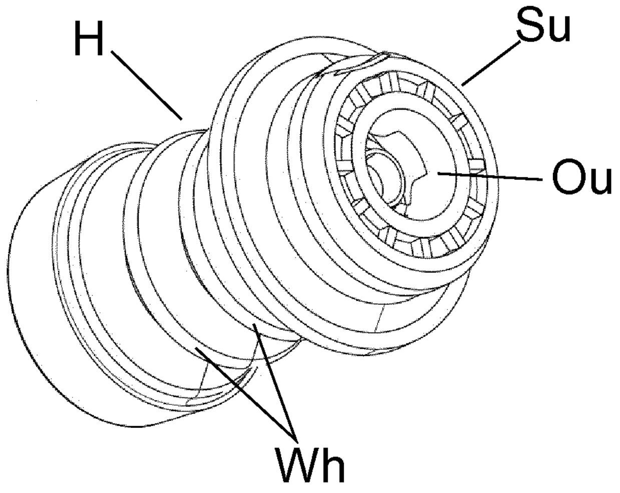


Fig. 1 c

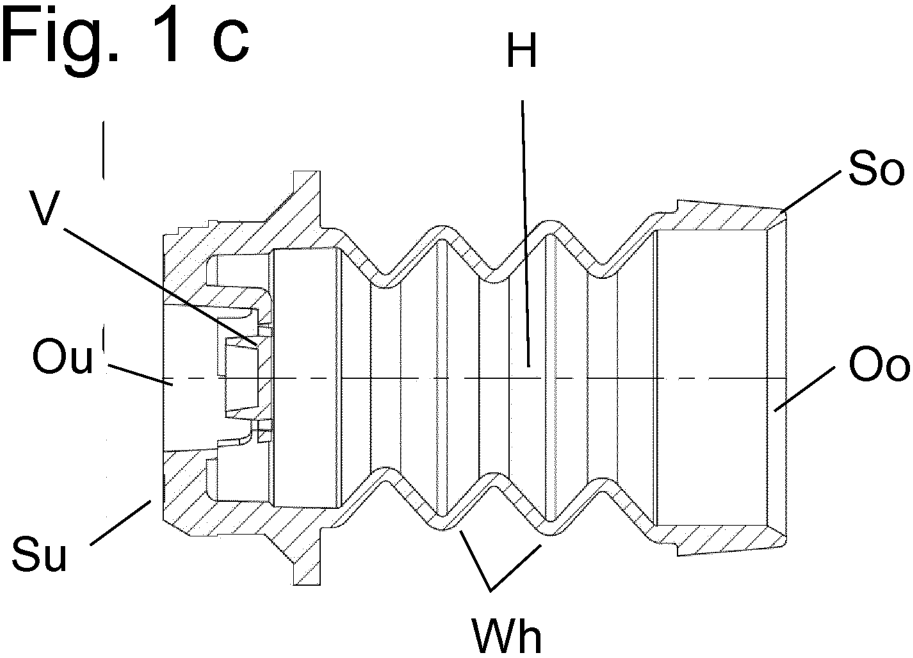

Fig. 1 d

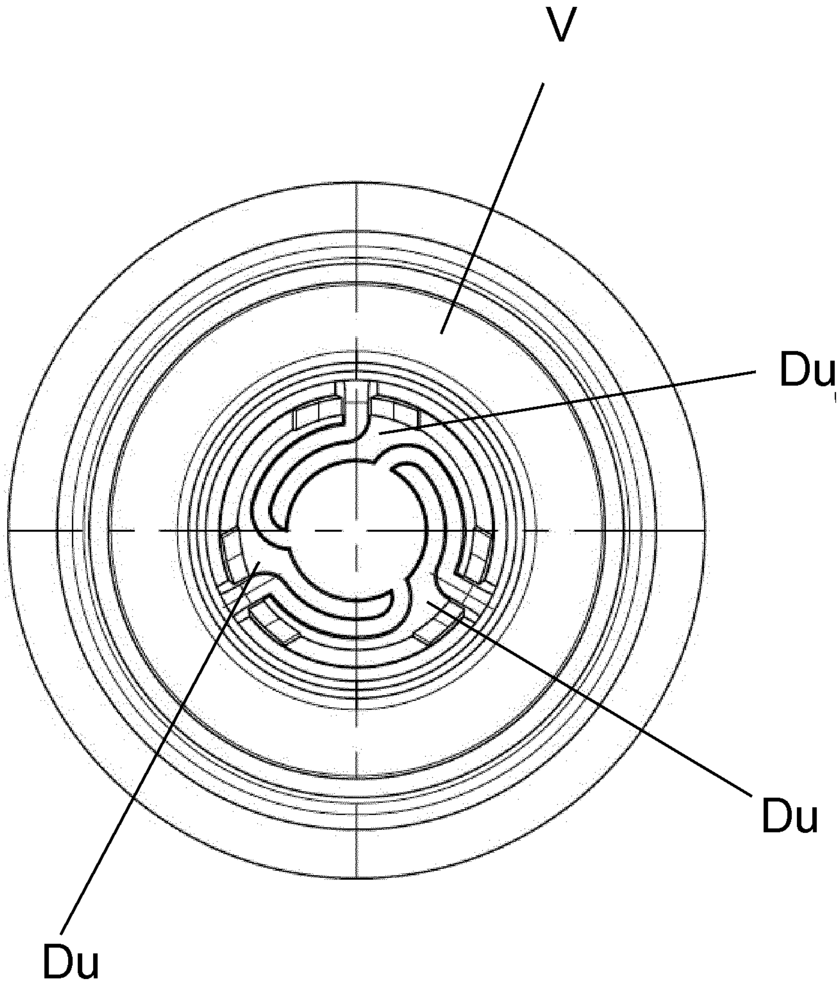

Fig. 2

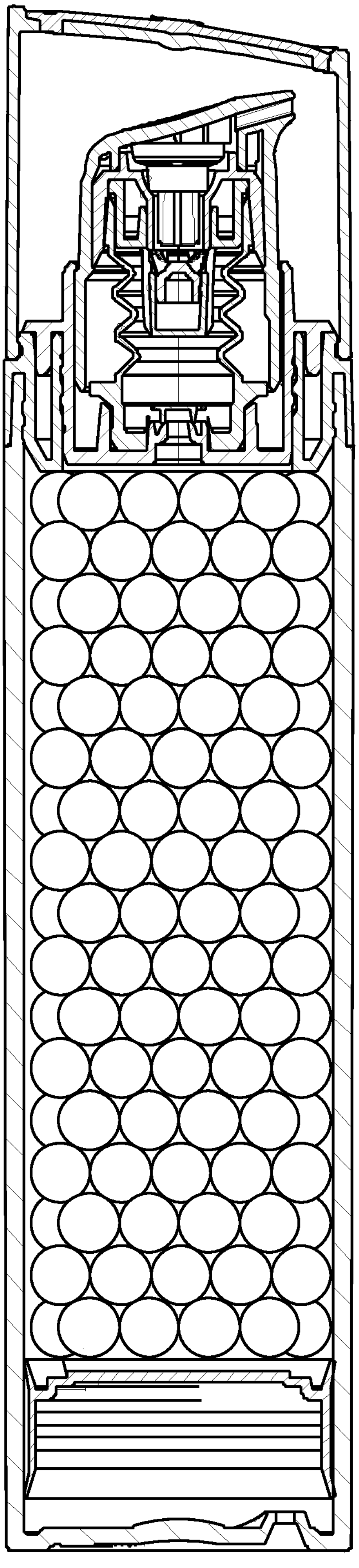

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente

- EP 2708264 A2
- US 4144325 A
- US 4248861 A
- DE 3309850 A
- JP 58180410 B
- DE 4424998 A1
- DE 4424998

### In der Beschreibung aufgeführte Nicht-Patentliteratur

- *Int. J. Cosm. Science,* 1988, vol. 10, 53
- **A. VOELCKEL et al.** *Zentralblatt Haut- und Geschlechtskrankheiten,* 1989, vol. 156, 2
- Skin Diseases Associated with Oxidative Injury. Oxidative Stress in Dermatology. Marcel Decker Inc, 323 ff
- *CHEMICAL ABSTRACTS,* 11138-66-2
- *CHEMICAL ABSTRACTS,* 58374-69-9
- *CHEMICAL ABSTRACTS,* 1333-84-2
- *CHEMICAL ABSTRACTS,* 7631-86-9
- *CHEMICAL ABSTRACTS,* 70356-09-1
- *CHEMICAL ABSTRACTS,* 180898-37-7
- *CHEMICAL ABSTRACTS,* 27503-81-7
- *CHEMICAL ABSTRACTS,* 90457-82-2
- *CHEMICAL ABSTRACTS,* 155633-54-8
- *CHEMICAL ABSTRACTS,* 178463-23-5